# EUROPEAN PATENT APPLICATION

(11) **EP 4 295 839 A1**
(43) Date of publication of application: **27.12.2023**
(21) Application number: 22179833.3
(22) Date of filing: 20.06.2022
(51) Int. Cl.: A61K 9/24, A61K 31/404, A61K 31/41

(54) **COMBINATION OF VALSARTAN AND INDAPAMIDE**

(71) Applicant: KRKA, d.d., Novo mesto, 8000 Novo mesto (SI)
(72) Inventor: The designation of the inventor has not yet been filed
(74) Representative: Kutzenberger Wolff & Partner

(57) **Abstract**

The invention relates to an oral pharmaceutical dosage form, preferably a bilayer tablet comprising a first layer and a second layer, wherein the pharmaceutical dosage form comprises (i) valsartan or a physiologically acceptable salt thereof; preferably wherein the pharmaceutical dosage form provides conventional release of valsartan or the physiologically acceptable salt thereof; and (ii) indapamide or a physiologically acceptable salt thereof; preferably wherein the pharmaceutical dosage form provides modified release of indapamide or the physiologically acceptable salt thereof. The invention further relates to a process for preparing such pharmaceutical dosage form.

## Description

The invention relates to an oral pharmaceutical dosage form, preferably a bilayer tablet comprising a first layer and a second layer, wherein the pharmaceutical dosage form comprises (i) valsartan or a physiologically acceptable salt thereof; preferably wherein the pharmaceutical dosage form provides conventional release of valsartan or the physiologically acceptable salt thereof; and (ii) indapamide or a physiologically acceptable salt thereof; preferably wherein the pharmaceutical dosage form provides modified release of indapamide or the physiologically acceptable salt thereof. The invention further relates to a process for preparing such pharmaceutical dosage form.

Valsartan ((*S*)-3-methyl-2-(*N*-1[2'-(2*H*-1,2,3,4-tetrazol-5-yl)biphenyl-4-yl]methyl}pentanamido)butanoic acid) is an angiotensin II receptor antagonist developed for the treatment of hypertension. Pharmaceutical compositions and dosage forms comprising valsartan are already known from e.g. WO 1997/049394 A2, WO 2005/089720 A1, WO 2005/082329 A2, WO 2000/038676 A1, WO 2005/041941 A2 and WO 2001/097805 A2.

Indapamide (4-chloro-N-(2-methylindolyn-1-yl)-3-sulfamoyl-benzamide) is a thiazide diuretic indicated in the treatment of hypertension and edema. Indapamide is known from e.g. US 3,565,911 A and US 5,334,392 A. Various processes for the preparation of indapamide and for pharmaceutical compositions comprising indapamide are also known from the prior art. Indapamide in a modified release form already exists on the market as a mono-drug product.

Several approaches to produce dosage forms comprising an angiotensin II receptor antagonist in combination with a diuretic are known from the prior art.

EP 2 260 833 A2 discloses the preparation of separate film-coated tablets comprising telmisartan and a diuretic, respectively, in such a size and shape that they can be filled into a capsule. By dividing the doses into two to four small tablets for telmisartan and into one or two small tablets for diuretic, a capsule of size 1 to 0 long can be filled. Yet, with this approach the drug dissolution rate of telmisartan was reduced compared to the single entities due to a lag-time effect of the large capsule shells. Furthermore, with regard to patients' compliance a 0 long capsule is not deemed reliable.

WO 2011/149438 A1 discloses a combination of antihypertensive active agents; a) indapamide (thiazide diuretic), b) amlodipine (calcium channel blocker) and c) telmisartan (angiotensin II receptor antagonist). Indapamide is present in both the immediate release and prolonged release layer. The prolonged release layer is surrounded by the immediate release layer. No specific examples are presented.

EP 2 252 273 B1 discloses a solid pharmaceutical composition comprising at least two layers, wherein the first layer comprises a non-peptide angiotensin II receptor antagonist or a pharmaceutically acceptable salt thereof in a dissolving matrix, and the second layer comprises a diuretic or a pharmaceutically acceptable salt thereof.

EP 1 467 712 B1 discloses a method of producing a bilayer tablet comprising the angiotensin II receptor antagonist telmisartan in combination with the diuretic hydrochlorothiazide (HCTZ). The combination drug displays an immediate drug release profile for both active ingredients.

Malykh T.V., Babkin A.P., Cardiovascular Therapy and Prevention, 2011;10(6):42-46 relates to effects of the fixed-dose combination therapy with indapamide and valsartan on renal function in patients with Type 2 diabetes mellitus and arterial hypertension.

Khamidullaeva G.A., Srojidinova N.Z., Abdullaeva G.J., Shakirova N.S., Khafizova L.S., Eurasian heart journal, 2014;(4):62-68 relates to indapamide and valsartan combined therapy in patients with arterial hypertension and metabolic disorders.

Nagay AV, Khamidullaeva GA, Srojidiniva NZ, Diabetes Complications, 2017; 1-4, relates to efficacy of triple combination antihypertensive therapy with valsartan, indapamide and amlodipine in hypertensive patients with pre-diabetes.

EMA/PDCO/671496/2012 is an opinion of the pediatric committee on the granting of a product specific waiver (EMEA-001377-PIP01-12) relating to a not further specified film-coated prolonged-release tablet containing valsartan and indapamide for treating essential hypertension, acute myocardial infarction, or heart failure in pediatric patients.

It is an object of the invention to provide a pharmaceutical dosage form having advantages compared to the prior art. The dosage form should preferably provide conventional release of valsartan and/or modified release of indapamide. Further, the dosage form should preferably display the same dissolution profile as mono-products of valsartan and indapamide. Still further, the dosage form should provide a satisfactory shelf-life/storage stability and patient compliance.

This object has been achieved by the subject-matter of the patent claims.

It has been surprisingly found that oral pharmaceutical dosage forms comprising valsartan or a physiologically acceptable salt thereof; preferably with conventional release thereof; and indapamide or a physiologically acceptable salt thereof; preferably with modified release thereof; provide several advantages.

First of all, coadministration of valsartan and indapamide has therapeutic advantages. Combination therapy of valsartan with indapamide, wherein indapamide is in a modified release form, shows synergistic therapeutic efficacy in the treatment of hypertension. Fixed-dose combinations of valsartan and indapamide allow for reducing administered dosages and/or administration frequencies, especially upon oral administration. The advantages for the patients are improved convenience, better assurance of compliance, reduction of severity and frequency of side effects, because such combinations maintain substantially constant blood levels and avoid the fluctuations associated with conventional formulations that are administered more than once a day.

Further, it has been found that advantageous pharmaceutical dosage forms, preferably bilayer tablets, can be prepared comprising valsartan and indapamide. Indapamide is sensitive to acidic and basic conditions. According to the present invention, valsartan is therefore preferably contained in a first unit, more preferably a first layer, and indapamide is preferably contained in a second unit that is spatially separated from the first unit, more preferably a second layer. With such oral pharmaceutical dosage form, stability problems are overcome that are otherwise caused by the incompatibility of indapamide, which is not stable in an acidic environment, with valsartan being an acid.

Further, it has been surprisingly found that such pharmaceutical dosage forms, preferably bilayer tablets, can provide conventional release of valsartan and modified release of indapamide. Preferably, the first layer of such a bilayer tablet contains valsartan and provides conventional release of valsartan and the second layer of such a bilayer tablet contains indapamide and provides modified release of indapamide. Modified release of indapamide can be achieved by embedding indapamide in a modified release matrix comprising a modified release matrix material.

Still further, it has been found that adequate dissolution depends on appropriate shape of the dosage form, preferably bilayer tablet, as well as the composition of the modified release matrix. Thus, both layers of such a bilayer tablet need to be comparatively thin to provide appropriate release profiles of both active ingredients. In particular, it has been surprisingly found that appropriate modified release profiles of indapamide can be achieved even in such comparatively thin layers by addition of an acrylic polymer, such as a carbomer (poly(acrylic acid)), as modified release matrix material.

Furthermore, it has been found that advantageous pharmaceutical dosage forms can be prepared when valsartan and indapamide are first separately granulated, preferably compacted, and then mixed with one or more extragranular excipients. The two mixtures comprising the active ingredients are then preferably compressed into bilayer tablets. It has been surprisingly found that adequate release profiles can be achieved regardless of the granulating process. In particular, no differences in dissolution of indapamide were observed for wet granulated or dry granulated indapamide.

Unless expressly stated otherwise, all percentages are expressed as weight percent. Unless expressly stated otherwise, all percentages are based on the total weight of the pharmaceutical dosage form according to the invention.

For the purpose of the specification, unless expressly stated otherwise, "*essentially consisting of*" or "*essentially the total amount*" means at least 95 wt.-%, preferably at least 98 wt.-%, more preferably at least 99 wt.-%.

Unless expressly stated otherwise, all references to USP, Ph. Eur., ASTM, EN ISO and the like refer to the official version that is valid on May 1, 2022.

Bilayer tablets are known to the skilled person. For details, reference is made to e.g. A. Abe-be et al., Review of bilayer tablet technology, Int J Pharm. 2014 Jan 30;461(1-2):549-58. doi: 10.1016/j.ijpharm.2013.12.028.

A first aspect of the invention relates to an oral pharmaceutical dosage form, preferably a bilayer tablet comprising a first layer and a second layer, comprising (i) valsartan or a physiologically acceptable salt thereof; preferably wherein the pharmaceutical dosage form provides conventional release of valsartan or the physiologically acceptable salt thereof; and (ii) indapamide or a physiologically acceptable salt thereof; preferably wherein the pharmaceutical dosage form provides modified release of indapamide or the physiologically acceptable salt thereof. The invention further relates to a process for preparing such pharmaceutical dosage form.

The term "*valsartan*" as used herein designates valsartan in its any known form or any polymorph form known from the state of the art. Valsartan used in the pharmaceutical dosage form according to the invention may be prepared according to any manufacturing process known from the state of the art.

In preferred embodiments of the invention, the valsartan is present in the non-salt form of valsartan.

Preferably, the content of valsartan or the physiologically acceptable salt thereof is within the range of 24±22 wt.-%, preferably 24±20 wt.-%, more preferably 24±18 wt.-%, still more preferably 24±16 wt.-%, yet more preferably 24±14 wt.-%, even more preferably 24±12 wt.-%, most preferably 24±10 wt.-%, and in particular 24±8.0 wt.-%; in each case relative to the total weight of the dosage form and expressed as equivalent weight relative to the non-salt form of valsartan.

Preferably, the content of valsartan or the physiologically acceptable salt thereof is within the range of 40±28 wt.-%, preferably 40±26 wt.-%, more preferably 40±24 wt.-%, still more preferably 40±22 wt.-%, yet more preferably 40±20 wt.-%, even more preferably 40±18 wt.-%, most preferably 40±16 wt.-%, and in particular 40±14 wt.-%; in each case relative to the total weight of the first layer and expressed as equivalent weight relative to the non-salt form of valsartan.

In preferred embodiments, the content of valsartan or the physiologically acceptable salt thereof is within the range of 16±15 wt.-%, preferably 16±14 wt.-%, more preferably 16±12 wt.-%, still more preferably 16±10 wt.-%, yet more preferably 16±8.0 wt.-%, even more preferably 16±6.0 wt.-%, most preferably 16±4.0 wt.-%, and in particular 16±2.0 wt.-%; in each case relative to the total weight of the dosage form and expressed as equivalent weight relative to the non-salt form of valsartan.

In other preferred embodiments, the content of valsartan or the physiologically acceptable salt thereof is within the range of 32±15 wt.-%, preferably 32±14 wt.-%, more preferably 32±12 wt.-%, still more preferably 32±10 wt.-%, yet more preferably 32±8.0 wt.-%, even more preferably 32±6.0 wt.-%, most preferably 32±4.0 wt.-%, and in particular 32±2.0 wt.-%; in each case relative to the total weight of the dosage form and expressed as equivalent weight relative to the non-salt form of valsartan.

In preferred embodiments, the dose of valsartan or the physiologically acceptable salt thereof is within the range of 120±1 10 mg, preferably 120±100 mg, more preferably 120±90 mg, still more preferably 120±80 mg, yet more preferably 120±70 mg, even more preferably 120±60 mg, most preferably 120±50 mg, and in particular 120±40 mg; in each case expressed as equivalent weight of the non-salt form of valsartan.

The term "*indapamide*" as used herein designates indapamide in its any known form or any polymorph form known from the state of the art. Indapamide used in the pharmaceutical dosage form according to the invention may be prepared according to any manufacturing process known from the state of the art.

In preferred embodiments of the invention, the indapamide is present in the non-salt form of indapamide.

Preferably, the content of indapamide or the physiologically acceptable salt thereof is within the range of 0.3±0.275 wt.-%, preferably 0.3±0.2 wt.-%, more preferably 0.3±0.175 wt.-%, still more preferably 0.3±0.15 wt.-%, yet more preferably 0.3±0.125 wt.-%, even more preferably 0.3±0.1 wt.-%, most preferably 0.3±0.075 wt.-%, and in particular 0.3±0.05 wt.-%; in each case relative to the total weight of the dosage form and expressed as equivalent weight relative to the non-salt form of indapamide.

Preferably, the content of indapamide or the physiologically acceptable salt thereof is within the range of 0.75±0.275 wt.-%, preferably 0.75±0.2 wt.-%, more preferably 0.75±0.175 wt.-%, still more preferably 0.75±0.15 wt.-%, yet more preferably 0.75±0.125 wt.-%, even more preferably 0.75±0.1 wt.-%, most preferably 0.75±0.075 wt.-%, and in particular 0.75±0.05 wt.-%; in each case relative to the total weight of the second layer and expressed as equivalent weight relative to the non-salt form of indapamide.

In preferred embodiments, the dose of indapamide or the physiologically acceptable salt thereof is within the range of 1.5±1.4 mg, preferably 1.5±1.2 mg, more preferably 1.5±1.0 mg, still more preferably 1.5±0.8 mg, yet more preferably 1.5±0.6 mg, even more preferably 1.5±0.4 mg, most preferably 1.5±0.2 mg, and in particular 1.5±0.1 mg; in each case expressed as equivalent weight of the non-salt form of indapamide.

In preferred embodiments, the weight ratio of valsartan or the physiologically acceptable salt thereof to indapamide or the physiologically acceptable salt thereof is within the range of from 124:1.0 to 38:1.0, preferably 120:1.0 to 42:1.0, more preferably 116:1.0 to 46:1.0, still more preferably 112:1.0 to 50:1.0, and yet more preferably 107:1.0 to 53:1.0; in each case expressed as equivalent weight of the non-salt form of valsartan and indapamide.

Preferably, the pharmaceutical dosage form according to the invention does not contain any other pharmaceutically active ingredients besides valsartan or the physiologically acceptable salt thereof and indapamide or the physiologically acceptable salt thereof.

Preferably, the oral pharmaceutical dosage form according to the invention provides conventional release of valsartan or the physiologically acceptable salt thereof.

The term *"conventional release"* (formerly referred to as "*immediate release*") refers to fast dissolution characteristics such that the active ingredient valsartan readily dissolves in a physiological aqueous medium. Preferably, *"conventional release"* is synonymous to "*immediate release*". Preferably, *"conventional release"* means that in an *in vitro* dissolution test according to Ph. Eur., basket method (40 mesh), 100 rpm, in 900 mL pH 6.8 phosphate buffer and at 37.0°C±0.5°C after 45 minutes at least 75 wt.-% of the active ingredient have been dissolved (released), relative to the total amount of this active ingredient that was originally contained in the dosage form (bilayer tablet). This definition of *"conventional release"* is in accordance with e.g. Ph. Eur. 5.17.1. "*Recommendations on dissolution testing*"*.*

Preferably, the oral pharmaceutical dosage form according to the invention provides an *in vitro* dissolution profile using USP I method (Apparatus 1 or Basket apparatus) with baskets 40 mesh at 100 rpm in 900 mL phosphate buffer at pH 6.8 and 37.0°C±0.5°C such that after 15 minutes at least 50 wt.-% of the valsartan that was initially contained in the pharmaceutical dosage form have been released; preferably at least 55 wt.-%, more preferably at least 60 wt.-%, still more preferably at least 65 wt.-%, yet more preferably at least 70 wt.-%, even more preferably at least 75 wt.-%, most preferably at least 80 wt.-%, and in particular at least 85 wt.-%.

Preferably, the oral pharmaceutical dosage form according to the invention provides modified release of indapamide or the physiologically acceptable salt thereof; preferably prolonged release (retarded release, sustained release), delayed release, or extended release; more preferably prolonged release.

The term "*modified release*" refers to release characteristics such that that the active ingredient indapamide is gradually released over time, allowing for a sustained effect.

Preferably, the oral pharmaceutical dosage form according to the invention provides an *in vitro* dissolution profile using USP II method (Apparatus 2 or paddles), at 100 rpm (tablets in Japanese sinkers (JS)) in 900 mL phosphate buffer at pH 6.8 and 37.0°C±0.5°C such that after 2 hours at most 70 wt.-% of the indapamide that was initially contained in the pharmaceutical dosage form have been released; preferably at most 65 wt.-%, more preferably at most 60 wt.-%, still more preferably at most 55 wt.-%, yet more preferably at most 50 wt.-%, even more preferably at most 45 wt.-%, most preferably at most 40 wt.-%, and in particular at most 35 wt.-%.

Preferably, indapamide is embedded in a modified release matrix comprising a modified release matrix material.

The term "*modified release matrix*" refers to a formulation where the active ingredient is embedded in a material that causes modified release (prolonged release) of this active ingredient from the formulation.

Preferably, the modified release matrix comprises or essentially consists of at least one polymer selected from the group consisting of
- cellulose ethers; preferably hydroxypropyl cellulose (HPC), hydroxypropyl methyl cellulose (HPMC; hypromellose), hydroxyethyl cellulose (HEC), methyl cellulose (MC), ethyl cellulose (EC), sodium carboxymethyl cellulose and carboxymethyl cellulose;
- cellulose esters; preferably cellulose acetate, cellulose acetate succinate, cellulose acetate phthalate, cellulose acetate propionate, cellulose acetate butyrate;
- acrylic polymers; preferably selected from cross-linked acrylic homopolymers, cross-linked acrylic copolymers, linear acrylic homopolymers, linear acrylic copolymers, cross-linked methacrylic homopolymers, cross-linked methacrylic copolymers, linear methacrylic homopolymers, and linear methacrylic copolymers; preferably selected from poly(acrylic acid), copolymers of acrylic acid, poly(acrylate), acrylate copolymers, poly(methyl acrylate), methyl acrylate copolymers, poly(ethoxyethyl acrylate), ethoxyethyl acrylate copolymers, poly(cyanoethyl acrylate), cyanoethyl acrylate copolymers, poly(aminoalkyl acrylate), aminoalkyl acrylate copolymers, poly(acrylic acid, acrylic acid alkylamide), poly(methacrylic acid, acrylic acid alkylamide), poly(hydroxyethyl acrylate), hydroxyethyl acrylate copolymers, poly(acrylic acid anhydride), acrylic acid anhydride copolymers, poly(acrylamide), acrylamide copolymers, poly(glycidyl acrylate), glycidyl acrylate copolymers, poly(methacrylic acid), methacrylic acid copolymers, poly(methacrylate), methacrylate copolymers, poly(methyl methacrylate), methyl methacrylate copolymers, poly(ethoxyethyl methacrylate), ethoxyethyl methacrylate copolymers, poly(cyanoethyl methacrylate), cyanoethyl methacrylate copolymers, poly(aminoalkyl methacrylate), aminoalkyl methacrylate copolymers, poly(acrylic acid, methacrylic acid alkylamide), poly(methacrylic acid, methacrylic acid alkylamide), poly(hydroxyethyl methacrylate), hydroxyethyl methacrylate copolymers, poly(methacrylic acid anhydride), methacrylic acid anhydride copolymers, poly(methacrylamide), methacrylamide copolymers, poly(glycidyl methacrylate), and glycidyl methacrylate copolymers;
- polysaccharides or gums; preferably xanthan gum, guar gum, karaya gum, locust bean gum, sodium alginate, carob gum, chitosan, polysaccharides of mannose and galactose, pectin, tragacanth, and agar-agar;
- polyalkylene glycols and polyalkylene oxides;
- polyvinylalcohol (PVA), cross-linked polyvinylalcohol (PVA), polyvinylpyrrolidone (PVP), cross-linked polyvinylpyrrolidone (PVP), polyvinylpyrrolidone-vinylacetate copolymers, polyvinylchloride (PVC), polyethylene vinyl acetate (PVA), polydimethylsiloxane (PDS), polyether urethane (PEU), polylactic acid (PLA), polyglycolic acid (PGA), polycaprolactone (PCL), polyanhydrides, polyorthoesters; and
- protein derived materials;
preferably selected from cellulose ethers and acrylic polymers.

Preferably, the modified release matrix material comprises or essentially consists of at least one cellulose ether, at least one acrylic polymer, or a mixture of at least one cellulose ether with at least one acrylic polymer.

Preferably, the modified release matrix material comprises or essentially consists of a first cellulose ether, a second cellulose ether, and/or an acrylic polymer.

In preferred embodiments, the modified release matrix material comprises or essentially consists of the first cellulose ether.

Preferably, the modified release matrix material comprises or essentially consists of two polymers.

In preferred embodiments, the modified release matrix material comprises or essentially consists of the first cellulose ether and the second cellulose ether.

In other preferred embodiments, the modified release matrix material comprises or essentially consists of the first cellulose ether and the acrylic polymer.

Preferably, the modified release matrix material comprises or essentially consists of three polymers.

Preferably, the modified release matrix material comprises or essentially consists of the first cellulose ether, the second cellulose ether and the acrylic polymer.

Preferably, the first cellulose ether and the second cellulose ether are independently of one another selected from hydroxypropyl cellulose (HPC), hydroxypropyl methyl cellulose (HPMC) and hydroxyethyl cellulose (HEC); preferably hydroxypropyl methyl cellulose (HPMC).

Preferably, the first cellulose ether differs from the second cellulose ether; preferably the first cellulose ether and the second cellulose ether are derived from the same monomers but differ in at least one parameter selected from the group consisting of solubility, viscosity and molecular weight.

In preferred embodiments, the first cellulose ether has a viscosity at 20°C determined in accordance with ASTM D2363 (or Ph. Eur.) within the range of from 750 to 8,500 mPa·s, preferably from 1,000 to 8,000 mPa·s, more preferably from 1,250 to 7,500 mPa·s, still more preferably from 1,500 to 7,000 mPa·s, yet more preferably 1,750 to 6,500 mPa·s, even more preferably from 2,000 to 6,000 mPa·s, most preferably from 2,250 to 5,500 mPa·s, and in particular from 2,500 to 5,000 mPa·s.

In preferred embodiments, the second cellulose ether has a viscosity at 20°C determined in accordance with ASTM D2363 (or Ph. Eur.) within the range of from 5,000 to 210,000 mPa·s, preferably from 15,000 to 200,000 mPa·s, more preferably from 25,000 to 190,000 mPa·s, still more preferably from 35,000 to 180,000 mPa·s, yet more preferably 45,000 to 170,000 mPa·s, even more preferably from 55,000 to 160,000 mPa·s, most preferably from 65,000 to 150,000 mPa·s, and in particular from 75,000 to 140,000 mPa·s.

Exemplary cellulose ethers are commercially available as HPMC K100 LV, HPMC K100M and HPMC K4M, e.g. under the tradename benecel^{™}. These hydroxypropyl methyl celluloses are of substitution type 2208 (K type) and differ in their viscosities measured in accordance with Ph. Eur. at a solution concentration of 2% (HPMC K100 LV: ~100 mPa·s (specification 80-120 mPa·s, e.g. benecel^{™} K100LV); HPMC K100M: ~100,000 mPa·s (specification 75,000-140,000 mPa·s, e.g. benecel^{™} K100M); HPMC K4M: ~4000 mPa·s (specification 2,700-5,040 mPa·s, e.g. benecel^{™} K4M)).

Preferably, the acrylic polymer is a polymer of acrylic acid; preferably a carbomer (poly(acrylic acid, preferably crosslinked), e.g. selected from carbomer types A, B and C according to USP). Exemplary acrylic polymers are commercially available as Carbopol^{®} and Pemulen^{®}.

Preferably, the total content of the modified release matrix material is within the range of 21±19 wt.-%, preferably 19±17 wt.-%, more preferably 17±15 wt.-%, still more preferably 15±13 wt.-%, yet more preferably 15±11 wt.-%, even more preferably 15±9.0 wt.-%, most preferably 15±7.0 wt.-%, and in particular 15±5.0 wt.-%; in each case relative to the total weight of the dosage form and based upon the total weight of all modified release matrix material that is contained in the dosage form.

Preferably, the total content of the modified release matrix material is within the range of 37±19 wt.-%, preferably 37±17 wt.-%, more preferably 37±15 wt.-%, still more preferably 37±13 wt.-%, yet more preferably 37±11 wt.-%, even more preferably 37±9.0 wt.-%, most preferably 37±7.0 wt.-%, and in particular 37±5.0 wt.-%; in each case relative to the total weight of the second layer and based upon the total weight of all modified release matrix material that is contained in the second layer.

Preferably, the total content of the first cellulose ether is within the range of 20±18 wt.-%, preferably 17±15 wt.-%, more preferably 13±12 wt.-%, still more preferably 13±10 wt.-%, yet more preferably 13±8.0 wt.-%, even more preferably 13±6.0 wt.-%, most preferably 13±4.0 wt.-%, and in particular 13±2.0 wt.-%; in each case relative to the total weight of the dosage form and based upon the total weight of all first cellulose ether that is contained in the dosage form.

Preferably, the total content of the first cellulose ether is within the range of 32±18 wt.-%, preferably 32±15 wt.-%, more preferably 32±12 wt.-%, still more preferably 32±10 wt.-%, yet more preferably 32±8.0 wt.-%, even more preferably 32±6.0 wt.-%, most preferably 32±4.0 wt.-%, and in particular 32±2.0 wt.-%; in each case relative to the total weight of the second layer and based upon the total weight of all first cellulose ether that is contained in the second layer.

Preferably, the total content of the second cellulose ether is within the range of 10±9.0 wt.-%, preferably 9.0±8.0 wt.-%, more preferably 8.0±7.0 wt.-%, still more preferably 7.0±6.0 wt.-%, yet more preferably 6.0±5.0 wt.-%, even more preferably 6.0±4.0 wt.-%, most preferably 6.0±3.0 wt.-%, and in particular 6.0±2.0 wt.-%; in each case relative to the total weight of the dosage form and based upon the total weight of all second cellulose ether that is contained in the dosage form.

Preferably, the total content of the second cellulose ether is within the range of 10±9.0 wt.-%, preferably 10±8.0 wt.-%, more preferably 10±7.0 wt.-%, still more preferably 10±6.0 wt.-%, yet more preferably 10±5.0 wt.-%, even more preferably 10±4.0 wt.-%, most preferably 10±3.0 wt.-%, and in particular 10±2.0 wt.-%; in each case relative to the total weight of the second layer and based upon the total weight of all second cellulose ether that is contained in the second layer.

Preferably, the total content of the acrylic polymer is within the range of 5.0±4.5 wt.-%, preferably 4.5±4.0 wt.-%, more preferably 4.0±3.5 wt.-%, still more preferably 3.5±3.0 wt.-%, yet more preferably 3.0±2.5 wt.-%, even more preferably 2.5±2.0 wt.-%, most preferably 2.0±1.5 wt.-%, and in particular 2.0±1.0 wt.-%; in each case relative to the total weight of the dosage form and based upon the total weight of all acrylic polymer that is contained in the dosage form.

Preferably, the total content of the acrylic polymer is within the range of 8.0±4.5 wt.-%, preferably 7.5±4.0 wt.-%, more preferably 7.0±3.5 wt.-%, still more preferably 6.5±3.0 wt.-%, yet more preferably 6.0±2.5 wt.-%, even more preferably 5.5±2.0 wt.-%, most preferably 5.0±1.5 wt.-%, and in particular 4.0±1.0 wt.-%; in each case relative to the total weight of the second layer and based upon the total weight of all acrylic polymer that is contained in the second layer.

Preferably, the pharmaceutical dosage form according to the invention comprises one or more excipients independently of one another selected from diluents, disintegrants, glidants, lubricants, coloring agents, and binders. Preferably, the one or more excipients are contained in the pharmaceutical dosage form according to the invention additionally to the modified release matrix.

The pharmaceutical dosage form according to the invention comprises valsartan and indapamide as the active substances, preferably in separate layers, and one or more pharmaceutically acceptable excipients such as one or more diluents, one or more lubricants, one or more glidants, one or more disintegrants, one or more binders, and the like. The term "*excipient*" as used herein refers to any physiologically acceptable substance that has no therapeutic activity as such. Pharmaceutically acceptable excipients may for example be selected from diluents, lubricants, glidants, disintegrants and binders. In preferred embodiments, the pharmaceutical dosage form according to the invention may further comprise any other pharmaceutically acceptable excipients that can be selected among any known state of the art for solid dosage forms, as described e.g. in Remington: The Science and Practice of Pharmacy, Edited by Loyd V. Allen, Jr, Pharmaceutical Press, 22 Edition, 2012. Individual excipients may have polyfunctional properties in the pharmaceutical dosage form according to the invention, e.g. may exert both disintegrating and binding properties or both lubricating and gliding properties or may exert filling, binding and disintegrating properties.

Preferably, the pharmaceutical dosage form according to the invention comprises at least one disintegrant; preferably selected from the group consisting of crospovidone, starch, maize starch, native starch, pregelatinized starch, sodium starch glycolate, hydroxypropyl starch, microcrystalline cellulose, sodium and/or calcium salts of carboxymethyl cellulose, cross-linked carboxymethylcellulose (e.g. croscarmellose sodium and/or croscarmellose calcium), low substituted hydroxypropyl cellulose, methylcellulose, polacrilin potassium, docusate sodium, alginic acid, sodium and/or calcium alginate, agar, guar gum, chitosan, and combinations thereof; preferably crospovidone.

In preferred embodiments, the overall content of said at least one disintegrant is within the range of 5.5±5.0 wt.-%, preferably 5.0±4.5 wt.-%, more preferably 4.4±4.0 wt.-%, still more preferably 4.4±3.5 wt.-%, yet more preferably 4.4±3.0 wt.-%, even more preferably 4.4±2.5 wt.-%, most preferably 4.4±2.0 wt.-%, and in particular 4.4±1.5 wt.-%; in each case relative to the total weight of the dosage form and based upon the total weight of all disintegrants that are contained in the dosage form.

In preferred embodiments, the overall content of said at least one disintegrant is within the range of 7.4±6.5 wt.-%, preferably 7.4±6.0 wt.-%, more preferably 7.4±5.5 wt.-%, still more preferably 7.4±5.0 wt.-%, yet more preferably 7.4±4.5 wt.-%, even more preferably 7.4±4.0 wt.-%, most preferably 7.4±3.5 wt.-%, and in particular 7.4±3.0 wt.-%; in each case relative to the total weight of the first layer and based upon the total weight of all disintegrants that are contained in the first layer.

Preferably, the pharmaceutical dosage form according to the invention comprises at least one diluent; preferably selected from the group consisting of
- lactose; preferably lactose monohydrate, anhydrous lactose, amorphous lactose, crystalline lactose, and spray dried and/or granulated lactose;
- monosaccharides, disaccharides and oligosaccharides; preferably sucrose, glucose, fructose, dextrates, saccharose, raffinose, trehalose, and dextrin,
- sugar alcohols; preferably xylitol, mannitol, maltitol, isomalt, and sorbitol;
- cellulose or cellulose derivatives; preferably powdered cellulose, microcrystalline cellulose, and silicified microcrystalline cellulose;
- starch or starch derivatives; preferably low moisture starch, corn starch, pregelatinized starch, and low moisture pregelatinized starch; and
- combinations thereof;
preferably lactose, sugar alcohols, cellulose and combinations thereof; more preferably crystalline lactose, mannitol, microcrystalline cellulose and combinations thereof; still more preferably mannitol, microcrystalline cellulose and combinations thereof.

In preferred embodiments, the overall content of said at least one diluent is within the range of 50±47.5 wt.-%, preferably 50±45 wt.-%, more preferably 50±40 wt.-%, still more preferably 50±35 wt.-%, yet more preferably 50±30 wt.-%, even more preferably 50±25 wt.-%, most preferably 50±20 wt.-%, and in particular 50±15 wt.-%; in each case relative to the total weight of the dosage form and based upon the total weight of all diluents that are contained in the dosage form.

In preferred embodiments, the overall content of said at least one diluent is within the range of 50±47.5 wt.-%, preferably 50±45 wt.-%, more preferably 50±40 wt.-%, still more preferably 50±35 wt.-%, yet more preferably 50±30 wt.-%, even more preferably 50±25 wt.-%, most preferably 40±20 wt.-%, and in particular 40±17 wt.-%; in each case relative to the total weight of the first layer and based upon the total weight of all diluents that are contained in the first layer.

In preferred embodiments, the overall content of said at least one diluent is within the range of 50±47.5 wt.-%, preferably 50±45 wt.-%, more preferably 50±40 wt.-%, still more preferably 50±35 wt.-%, yet more preferably 50±30 wt.-%, even more preferably 50±25 wt.-%, most preferably 50±20 wt.-%, and in particular 50±15 wt.-%; in each case relative to the total weight of the second layer and based upon the total weight of all diluents that are contained in the second layer.

Preferably, the pharmaceutical dosage form according to the invention comprises microcrystalline cellulose; more preferably a first type microcrystalline cellulose, a second type microcrystalline cellulose and/or a third type microcrystalline cellulose.

Preferably, the first type microcrystalline cellulose has an average particle size, preferably an average mean particle diameter (Z-average), within the range of 50±40 µm, preferably 50±35 µm, more preferably 50±30 µm, still more preferably 50±25 µm, yet more preferably 50±20 µm, even more preferably 50±15 µm, most preferably 50±10 µm, and in particular 50±5.0 µm; preferably determined in accordance with USP <429> *"Laser Diffraction Measurement of Particle Size".*

Preferably, the second type microcrystalline cellulose has an average particle size, preferably an average mean particle diameter (Z-average), within the range of 100±80 µm, preferably 100±70 µm, more preferably 100±60 µm, still more preferably 100±50 µm, yet more preferably 100±40 µm, even more preferably 100±30 µm, most preferably 100±20 µm, and in particular 100±10 µm; preferably determined in accordance with USP <429> *"Laser Diffraction Measurement of Particle Size".*

Preferably, the third type microcrystalline cellulose has
- a volume median diameter D90 (Dv90) within the range of from 225 to 530 µm, preferably from 250 to 505 µm, more preferably from 275 to 480 µm;
- a volume median diameter D50 (Dv50) within the range of from 102 to 320 µm, preferably 122 to 300 µm, more preferably 142 to 280 µm; and/or
- a volume median diameter D10 (Dv10) of at most 225 µm, preferably at most 200 µm, more preferably at most 175 µm;
preferably determined in accordance with USP <429> *"Laser Diffraction Measurement of Particle Size".*

Preferably, the pharmaceutical dosage form according to the invention comprises mannitol; more preferably a first type mannitol and/or a second type mannitol.

Preferably, the first type mannitol (e.g. Parteck^{®} M200) has an average particle size, preferably an average mean particle diameter (Z-average) or circular equivalent diameter (CEDc(vol)), within the range of 190±40 µm, preferably 190±35 µm, more preferably 190±30 µm, still more preferably 190±25 µm, yet more preferably 190±20 µm, even more preferably 190±15 µm, most preferably 190±10 µm, and in particular 190±5.0 µm; preferably determined in accordance with USP <429> "*Laser Diffraction Measurement of Particle Size".*

Preferably, the first type mannitol (e.g. Parteck^{®} M200) has a particle size distribution such that not more than 15 wt.-% pass a sieve having a mesh size of 53 µm and at least 15 wt.-% pass a sieve having a mesh size of 500 µm.

Preferably, the first type mannitol (e.g. Parteck^{®} M200) has
- a volume median diameter D90 (Dv90) within the range of from 280 to 400 µm, preferably from 300 to 380 µm, more preferably from 320 to 360 µm;
- a volume median diameter D50 (Dv50) within the range of from 100 to 220 µm, preferably 130 to 190 µm, more preferably 150 to 170 µm; and/or
- a volume median diameter D10 (Dv10) within the range of from 20 to 120 µm, preferably 40 to 100 µm, more preferably 60 to 80 µm;
preferably determined in accordance with USP <429> *"Laser Diffraction Measurement of Particle Size".*

Preferably, the second type mannitol (e.g. Pearlitol^{®} 160C or Pearlitol^{®} 200 SD) has an average particle size, preferably an average mean particle diameter (Z-average) or circular equivalent diameter (CEDc(vol)), within the range of 170±55 µm, preferably 170±50 µm, more preferably 170±45 µm, still more preferably 170±40 µm, yet more preferably 170±35 µm, even more preferably 170±30 µm, most preferably 170±25 µm, and in particular 170±20 µm; preferably determined in accordance with USP *<429> "Laser Diffraction Measurement of Particle Size".*

Preferably, the second type mannitol (e.g. Pearlitol^{®} 160C or Pearlitol^{®} 200 SD) has
- a volume median diameter D90 (Dv90) within the range of from 150 to 400 µm, preferably from 200 to 350 µm, more preferably from 220 to 330 µm;
- a volume median diameter D50 (Dv50) within the range of from 70 to 250 µm, preferably 110 to 210 µm, more preferably 150 to 170 µm ; and/or
- a volume median diameter D10 (Dv10) within the range of from 20 to 140 µm, preferably 50 to 110 µm, more preferably 65 to 95 µm;
preferably determined in accordance with USP <429> *"Laser Diffraction Measurement of Particle Size".*

Preferably, the second type mannitol (e.g. Pearlitol^{®} 160C) has an average particle size, preferably an average mean particle diameter (Z-average) or circular equivalent diameter (CEDc(vol)), within the range of 160±40 µm, preferably 160±35 µm, more preferably 160±30 µm, still more preferably 160±25 µm, yet more preferably 160±20 µm, even more preferably 160±15 µm, most preferably 160±10 µm, and in particular 160±5 µm; preferably determined in accordance with USP <429> "*Laser Diffraction Measurement of Particle Size".*

In preferred embodiments, the second type mannitol (e.g. Pearlitol^{®} 160C) has
- a volume median diameter D90 (Dv90) within the range of from 315±120 µm, preferably from 315±80 µm, more preferably from 315±40 µm;
- a volume median diameter D50 (Dv50) within the range of from 160±60 µm, preferably 160±40 µm, more preferably 160±20 µm; and/or
- a volume median diameter D10 (Dv10) within the range of from 32±15 µm, preferably 32±10 µm, more preferably 32±5 µm;
preferably determined in accordance with USP <429> *"Laser Diffraction Measurement of Particle Size".*

Preferably, the second type mannitol (e.g. Pearlitol^{®} 200 SD) has an average particle size, preferably an average mean particle diameter (Z-average) or circular equivalent diameter (CEDc(vol)), within the range of 200±40 µm, preferably 200±35 µm, more preferably 200±30 µm, still more preferably 200±25 µm, yet more preferably 200±20 µm, even more preferably 200±15 µm, most preferably 200±10 µm, and in particular 200±5 µm; preferably determined in accordance with USP <429> "*Laser Diffraction Measurement of Particle Size".*

In preferred embodiments, the second type mannitol (e.g. Pearlitol^{®} 200 SD) has
- a volume median diameter D90 (Dv90) within the range of from 240±120 µm, preferably from 240±80 µm, more preferably from 240±40 µm ;
- a volume median diameter D50 (Dv50) within the range of from 150±60 µm, preferably 150±40 µm, more preferably 150±20 µm; and/or
- a volume median diameter D10 (Dv10) within the range of from 65±30 µm, preferably 65±20 µm, more preferably 65±10 µm;
preferably determined in accordance with USP <429> *"Laser Diffraction Measurement of Particle Size".*

For the purpose of the specification, the term median diameter or mean diameter refers to the volume median diameter or volume mean diameter of the particle size distribution. A skilled person recognizes that laser diffraction measurements provide volume based values. Values D30, D50 and D90 indicate that 30%, 50% and 90% of the particles by volume are smaller than the specified values, respectively. The volume median diameter can for example be determined by a laser diffraction method such as Malvern Master Sizer, preferably in accordance with USP <429> "*Laser Diffraction Measurement of Particle Size*". Suitable measuring conditions can be determined by routine experimentation and on the basis of commercially available samples that can be used for calibration.

For the purpose of the specification a diluent is considered equivalent to a filler. Thus, both excipients perform the same functions, namely to increase the weight and improve the uniformity of the contents.

Preferably, the pharmaceutical dosage form according to the invention comprises at least one glidant; preferably selected from the group consisting of colloidal silica, talc, magnesium trisilicate, and combinations thereof; preferably colloidal silica.

In preferred embodiments, the overall content of said at least one glidant is within the range of 2.5±2.0 wt.-%, preferably 2.0±1.75 wt.-%, more preferably 1.75±1.5 wt.-%, still more preferably 1.5±1.25 wt.-%, yet more preferably 1.25±1.0 wt.-%, even more preferably 1.25±0.75 wt.-%, most preferably 1.25±0.5 wt.-%, and in particular 1.25±0.25 wt.-%; in each case relative to the total weight of the dosage form and based upon the total weight of all glidants that are contained in the dosage form.

In preferred embodiments, the overall content of said at least one glidant is within the range of 2.5±2.0 wt.-%, preferably 2.0±1.75 wt.-%, more preferably 1.75±1.5 wt.-%, still more preferably 1.5±1.25 wt.-%, yet more preferably 1.5±1.0 wt.-%, even more preferably 1.5±0.75 wt.-%, and most preferably 1.5±0.5 wt.-%; in each case relative to the total weight of the first layer and based upon the total weight of all glidants that are contained in the first layer.

In preferred embodiments, the overall content of said at least one glidant is within the range of 2.5±2.0 wt.-%, preferably 2.0±1.75 wt.-%, more preferably 1.75±1.5 wt.-%, still more preferably 1.5±1.25 wt.-%, yet more preferably 1.5±1.0 wt.-%, even more preferably 1.5±0.75 wt.-%, most preferably 1.5±0.5 wt.-%, and in particular 1.5±0.25 wt.-%; in each case relative to the total weight of the second layer and based upon the total weight of all glidants that are contained in the second layer.

Preferably, the pharmaceutical dosage form according to the invention comprises at least one lubricant; preferably selected from stearic acid, magnesium stearate, calcium stearate, aluminum stearate, zinc stearate, magnesium palmitate, magnesium oleate, sodium stearyl fumarate, glycerol tribehenate and combinations thereof; preferably magnesium stearate.

In preferred embodiments, the overall content of said at least one lubricant is within the range of 2.0±1.8 wt.-%, preferably 1.8±1.6 wt.-%, more preferably 1.8±1.4 wt.-%, still more preferably 1.8±1.2 wt.-%, yet more preferably 1.8±1.0 wt.-%, even more preferably 1.8±0.8 wt.-%, most preferably 1.8±0.6 wt.-%, and in particular 1.8±0.4 wt.-%; in each case relative to the total weight of the dosage form and based upon the total weight of all lubricants that are contained in the dosage form.

In preferred embodiments, the overall content of said at least one lubricant is within the range of 2.0±1.8 wt.-%, preferably 2.0±1.6 wt.-%, more preferably 2.0±1.4 wt.-%, still more preferably 2.0±1.2 wt.-%, yet more preferably 2.0±1.0 wt.-%, even more preferably 2.0±0.8 wt.-%, most preferably 2.0±0.6 wt.-%, and in particular 2.0±0.5 wt.-%; in each case relative to the total weight of the first layer and based upon the total weight of all lubricants that are contained in the first layer.

In preferred embodiments, the overall content of said at least one lubricant is within the range of 2.0±1.8 wt.-%, preferably 1.8±1.6 wt.-%, more preferably 1.8±1.4 wt.-%, still more preferably 1. 8±1.2 wt.-%, yet more preferably 1.8±1.0 wt.-%, even more preferably 1.0±0.8 wt.-%, most preferably 1.0±0.6 wt.-%, and in particular 1.0±0.4 wt.-%; in each case relative to the total weight of the second layer and based upon the total weight of all lubricants that are contained in the second layer.

Preferably, the pharmaceutical dosage form according to the invention comprises at least one coloring agent; preferably selected from dyes and pigments such as iron oxide red, yellow, brown or black, titanium dioxide and combinations thereof; preferably iron oxide yellow or iron oxide red. In preferred embodiments, the coloring agent is iron oxide red and the overall weight content of valsartan or the physiologically acceptable salt thereof is within the range of 16±15 wt.-%, relative to the total weight of the dosage form and expressed as equivalent weight relative to the non-salt form of valsartan. In other preferred embodiments, the coloring agent is iron oxide yellow and the overall weight content of valsartan or the physiologically acceptable salt thereof is within the range of 32±15 wt.-%, relative to the total weight of the dosage form and expressed as equivalent weight relative to the non-salt form of valsartan.

In preferred embodiments, the overall content of said at least one coloring agent is within the range of 0.04±0.03 wt.-%, preferably 0.04±0.01 wt.-%; in each case relative to the total weight of the dosage form and based upon the total weight of all coloring agents that are contained in the dosage form.

Preferably, the pharmaceutical dosage form according to the invention comprises or essentially consists of
- valsartan or the physiologically acceptable salt thereof; preferably with a content within the range of 24±8.0 wt.-%; relative to the total weight of the dosage form and expressed as equivalent weight relative to the non-salt form of valsartan;
- indapamide or the physiologically acceptable salt thereof; preferably with a content within the range of 0.3±0.05 wt.-%; relative to the total weight of the dosage form and expressed as equivalent weight relative to the non-salt form of indapamide;
- a modified release matrix material; preferably comprising or essentially consisting of the first cellulose ether, the second cellulose ether, and/or the acrylic polymer; more preferably the first cellulose ether and the acrylic polymer; still more preferably with a total content within the range of 15±5.0 wt.-%; relative to the total weight of the dosage form and based upon the total weight of all modified release matrix material that is contained in the dosage form;
- at least one disintegrant; preferably crospovidone; more preferably with an overall content within the range of 4.4±1.5 wt.-%; relative to the total weight of the dosage form and based upon the total weight of all disintegrants that are contained in the dosage form;
- at least one diluent; preferably crystalline lactose, mannitol, and/or microcrystalline cellulose; more preferably mannitol and microcrystalline cellulose; still more preferably with an overall content within the range of 50±15 wt.-%; relative to the total weight of the dosage form and based upon the total weight of all diluents that are contained in the dosage form;
- optionally, at least one glidant; preferably colloidal silica; more preferably with an overall content within the range of 1.25±0.25 wt.-%; relative to the total weight of the dosage form and based upon the total weight of all glidants that are contained in the dosage form; and
- optionally, at least one lubricant; preferably magnesium stearate; more preferably with an overall content within the range of 1.8±0.4 wt.-%; relative to the total weight of the dosage form and based upon the total weight of all lubricants that are contained in the dosage form.

Preferably, the pharmaceutical dosage form according to the invention is selected from the group consisting of tablets, pills, powders, lozenges, sachets, soft gelatin capsules, hard gelatin capsules, and suppositories; preferably tablets; more preferably bilayer tablets.

In preferred embodiments, the pharmaceutical dosage form according to the invention is coated; preferably with a water-soluble coating material.

Preferably, the overall weight of the pharmaceutical dosage form according to the invention is within the range of 500±200 mg, preferably 500±175 mg, more preferably 500±150 mg, still more preferably 500±125 mg, yet more preferably 500±100 mg, even more preferably 500±75 mg, most preferably 500±50 mg, and in particular 500±25 mg.

In preferred embodiments, the pharmaceutical dosage form according to the invention has a diameter of at least 3.0 mm, preferably at least 4.0 mm, more preferably at least 5.0 mm, still more preferably at least 6.0 mm, yet more preferably at least 7.0 mm, even more preferably at least 8.0 mm, most preferably at least 9.0 mm, and in particular at least 10 mm.

In preferred embodiments, the pharmaceutical dosage form according to the invention has a diameter of at most 17 mm, preferably at most 16 mm, more preferably at most 15 mm, still more preferably at most 14 mm, yet more preferably at most 13 mm, even more preferably at most 12 mm.

In preferred embodiments, the pharmaceutical dosage form according to the invention has a diameter within the range of 10±6.0 mm, preferably 10±4.0 mm, more preferably 10±2.0 mm.

Preferably, the pharmaceutical dosage form according to the invention has a thickness within the range of 5.0±4.0 mm, preferably 5.0±3.5 mm, more preferably 5.0±3.0 mm, still more preferably 5.0±2.5 mm, yet more preferably 5.0±2.0 mm, even more preferably 5.0±1.5 mm, most preferably 5.0±1.0 mm, and in particular 5.0±0.7 mm.

Preferably, the dimensions of the dosage form are determined using any technique known from the state of the art, as for example an optical microscope (e.g. stereomicroscope) or any other similar techniques known in the art.

Preferably, the pharmaceutical dosage form according to the invention comprises
- a first unit (preferably a first layer) that contains essentially the total amount of valsartan or the physiologically acceptable salt thereof; and
- a second unit (preferably a second layer) that is spatially separated from the first unit and that contains essentially the total amount of indapamide or the physiologically acceptable salt thereof.

In preferred embodiments, the relative weight ratio of the first unit (preferably the first layer) to the second unit (preferably the second layer) is from 4:1 to 1:1, preferably from 3:1 to 1:1, more preferably from 2:1 to 1:1.

In preferred embodiments, the content of the first unit (preferably the first layer) is within the range of 60±35 wt.-%, preferably 60±30 wt.-%, more preferably 60±25 wt.-%, still more preferably 60±20 wt.-%, yet more preferably 60±15 wt.-%, even more preferably 60±10 wt.-%, most preferably 60±5.0 wt.-%, and in particular 60±2.5 wt.-%; in each case relative to the total weight of the dosage form.

In preferred embodiments, the content of the second unit (preferably the second layer) is within the range of 40±35 wt.-%, preferably 40±30 wt.-%, more preferably 40±25 wt.-%, still more preferably 40±20 wt.-%, yet more preferably 40±15 wt.-%, even more preferably 40±10 wt.-%, most preferably 40±5.0 wt.-%, and in particular 40±2.5 wt.-%; in each case relative to the total weight of the dosage form.

Preferably, the dosage form is a bilayer tablet comprising a first layer (first unit) which comprises valsartan or the physiologically acceptable salt thereof and a second layer (second unit) which comprises indapamide or the physiologically acceptable salt thereof.

The term "*layer*" as used herein designates a layer in a multilayer tablet wherein at least two active substances are not in intimate contact and that they are separated from each other (except at the interface of the layers). The term "*layer*" refers to a physical part of the oral dosage form comprising at least two layers, wherein the components of one layer are not intimately mixed with the components of another layer of the composition. In particular, the interaction between the components of one layer and the components of another layer is reduced. A multilayer tablet according to the invention has at least 2 layers, for example, a bilayer tablet involves the compression of two formulations comprising an active substance into a single solid oral dosage form, while maintaining physical separation of the formulations by layering one on top of the other. According to the invention the layer is preferably formed from a compression mixture, which consists of at least one active substance and one or more pharmaceutically acceptable excipients. While it is contemplated that the dosage form according to the invention may be a multilayer tablet having more than two layers, the dosage form according to the invention preferably is a bilayer tablet.

The term "*compression mixture*" as used herein designates the mixture of at least one pharmaceutically active substance and one or more pharmaceutically acceptable excipients. Preferably, the compression mixture contains a granulate comprising the pharmaceutically active substance, optionally mixed with one or more extragranular excipients.

Preferably, the first layer comprises essentially the total amount of valsartan or the physiologically acceptable salt thereof that is contained in the pharmaceutical dosage form.

Preferably, the second layer comprises essentially the total amount of indapamide or the physiologically acceptable salt thereof that is contained in the pharmaceutical dosage form.

Preferably, the first layer comprises a first layer intragranular phase and a first layer extragranular phase.

The terms "*intragranular phase*" and "*extragranular phase*" are known to the skilled person and are used in the conventional meaning. As the valsartan layer and/or the indapamide layer is preferably prepared from a compression mixture that contains a granulate, optionally mixed with one or more extragranular excipients, the granulate and its constituents forms the intragranular phase, whereas the optionally present one or more extragranular excipients form the extragranular phase.

Preferably, valsartan or the physiologically acceptable salt thereof is contained in the first layer intragranular phase.

In preferred embodiments, the first layer, preferably the first layer intragranular phase, comprises at least one disintegrant as defined above.

In preferred embodiments, the first layer, preferably the first layer intragranular phase and the first layer extragranular phase, comprises at least one diluent as defined above; preferably microcrystalline cellulose.

In preferred embodiments, the first layer, preferably the first layer intragranular phase, comprises at least one glidant as defined above.

In preferred embodiments, the first layer, preferably the first layer intragranular phase and the first layer extragranular phase, comprises at least one lubricant as defined above.

Preferably, the first layer of the bilayer tablet according to the invention comprises or essentially consists of
- valsartan or the physiologically acceptable salt thereof; preferably with an content within the range of 24±8.0 wt.-%; relative to the total weight of the dosage form and expressed as equivalent weight relative to the non-salt form of valsartan;
- at least one disintegrant; preferably crospovidone; more preferably with an content within the range of 4.4±1.5 wt.-%; relative to the total weight of the dosage form and based upon the total weight of all disintegrants that are contained in the dosage form;
- at least one diluent; preferably microcrystalline cellulose; more preferably with an content within the range of 40±27 wt.-%, preferably 25±15 wt.-%; relative to the total weight of the dosage form and based upon the total weight of all diluents that are contained in the dosage form;
- optionally, at least one glidant; preferably colloidal silica; more preferably with an content within the range of 0.6±0.3 wt.-%; relative to the total weight of the dosage form and based upon the total weight of all glidants that are contained in the dosage form; and
- optionally, at least one lubricant; preferably magnesium stearate; more preferably with an content within the range of 1.4±0.5 wt.-%; relative to the total weight of the dosage form and based upon the total weight of all lubricants that are contained in the dosage form.

Preferably, the first layer intragranular phase of the bilayer tablet according to the invention comprises or essentially consists of
- valsartan or the physiologically acceptable salt thereof; preferably with an content within the range of 24±8.0 wt.-%; relative to the total weight of the dosage form and expressed as equivalent weight relative to the non-salt form of valsartan;
- at least one disintegrant; preferably crospovidone; more preferably with an content within the range of 4.4±1.5 wt.-%; relative to the total weight of the dosage form and based upon the total weight of all disintegrants that are contained in the dosage form;
- at least one diluent; preferably microcrystalline cellulose; more preferably the second type microcrystalline cellulose or the third type microcrystalline cellulose; still more preferably with an content within the range of 32±23 wt.-%, preferably 22±11 wt.-%; relative to the total weight of the dosage form and based upon the total weight of all diluents that are contained in the dosage form;
- optionally, at least one glidant; preferably colloidal silica; more preferably with an content within the range of 0.7±0.3 wt.-%; relative to the total weight of the dosage form and based upon the total weight of all glidants that are contained in the dosage form; and
- optionally, at least one lubricant; preferably magnesium stearate; more preferably with an content within the range of 0.7±0.3 wt.-%; relative to the total weight of the dosage form and based upon the total weight of all lubricants that are contained in the dosage form.

Preferably, the first layer extragranular phase of the bilayer tablet according to the invention comprises or essentially consists of
- at least one diluent; preferably microcrystalline cellulose; more preferably the second type microcrystalline cellulose; still more preferably with an content within the range of 9.0±5.0 wt.-%; relative to the total weight of the dosage form and based upon the total weight of all diluents that are contained in the dosage form; and
- optionally, at least one lubricant; preferably magnesium stearate; more preferably with an content within the range of 0.75±0.25 wt.-%; relative to the total weight of the dosage form and based upon the total weight of all lubricants that are contained in the dosage form.

Preferably, the second layer comprises a second layer intragranular phase and a second layer extragranular phase.

Preferably, indapamide or the physiologically acceptable salt thereof is contained in the second layer intragranular phase.

In preferred embodiments, the second layer, preferably the second layer intragranular phase and the second layer extragranular phase, comprises a modified release matrix material as defined above.

In preferred embodiments, the second layer, preferably the second layer intragranular phase and the second layer extragranular phase, comprises at least one diluent as defined above; preferably microcrystalline cellulose and crystalline lactose, or microcrystalline cellulose and mannitol; more preferably microcrystalline cellulose and mannitol; preferably the first type microcrystalline cellulose and/or the second type microcrystalline cellulose.

In preferred embodiments, the second layer, preferably the second layer intragranular phase and the second layer extragranular phase, comprises at least one glidant as defined above.

In preferred embodiments, the second layer, preferably the second layer intragranular phase and the second layer extragranular phase, comprises at least one lubricant as defined above.

Preferably, the second layer of the bilayer tablet according to the invention comprises or essentially consists of
- indapamide or the physiologically acceptable salt thereof; preferably with an content within the range of 0.3±0.05 wt.-%; relative to the total weight of the dosage form and expressed as equivalent weight relative to the non-salt form of indapamide;
- a modified release matrix material; preferably comprising or essentially consisting of the first cellulose ether, the second cellulose ether, and/or the acrylic polymer; more preferably the first cellulose ether and the acrylic polymer; still more preferably with an content within the range of 15±5.0 wt.-%; relative to the total weight of the dosage form and based upon the total weight of all modified release matrix material that is contained in the dosage form;
- at least one diluent; preferably crystalline lactose, mannitol, and/or microcrystalline cellulose; more preferably mannitol and microcrystalline cellulose; preferably the first type microcrystalline cellulose and/or the second type microcrystalline cellulose; still more preferably with an content within the range of 21±5.0 wt.-%; relative to the total weight of the dosage form and based upon the total weight of all diluents that are contained in the dosage form;
- optionally, at least one glidant; preferably colloidal silica; more preferably with an content within the range of 0.6±0.2 wt.-%; relative to the total weight of the dosage form and based upon the total weight of all glidants that are contained in the dosage form; and
- optionally, at least one lubricant; preferably magnesium stearate; more preferably with an content within the range of 0.4±0.2 wt.-%; relative to the total weight of the dosage form and based upon the total weight of all lubricants that are contained in the dosage form.

In preferred embodiments, the second layer intragranular phase comprises a modified release matrix material comprising or essentially consisting of the first cellulose ether.

In other preferred embodiments, the second layer intragranular phase comprises a modified release matrix material comprising or essentially consisting of the first cellulose ether and the second cellulose ether.

In preferred embodiments, the second layer intragranular phase of the bilayer tablet according to the invention comprises or essentially consists of
- indapamide or the physiologically acceptable salt thereof; preferably with an content within the range of 0.3±0.05 wt.-%; relative to the total weight of the dosage form and expressed as equivalent weight relative to the non-salt form of indapamide;
- a modified release matrix material; preferably comprising or essentially consisting of the first cellulose ether; more preferably with an content within the range of 14±6.0 wt.-%; relative to the total weight of the dosage form and based upon the total weight of all modified release matrix material that is contained in the dosage form;
- at least one diluent; preferably crystalline lactose and microcrystalline cellulose; preferably the first type microcrystalline cellulose; more preferably with an content within the range of 14±5.0 wt.-%; relative to the total weight of the dosage form and based upon the total weight of all diluents that are contained in the dosage form; and
- optionally, at least one glidant; preferably colloidal silica; more preferably with an content within the range of 0.4±0.2 wt.-%; relative to the total weight of the dosage form and based upon the total weight of all glidants that are contained in the dosage form.

In other preferred embodiments, the second layer intragranular phase of the bilayer tablet according to the invention comprises or essentially consists of
- indapamide or the physiologically acceptable salt thereof; preferably with an content within the range of 0.3±0.05 wt.-%; relative to the total weight of the dosage form and expressed as equivalent weight relative to the non-salt form of indapamide;
- a modified release matrix material; preferably comprising or essentially consisting of the first cellulose ether; more preferably with an content within the range of 9.0±2.0 wt.-%; relative to the total weight of the dosage form and based upon the total weight of all modified release matrix material that is contained in the dosage form;
- at least one diluent; preferably crystalline lactose, mannitol, and/or microcrystalline cellulose; more preferably mannitol and microcrystalline cellulose; preferably the first type microcrystalline cellulose and/or the second type microcrystalline cellulose; still more preferably with an content within the range of 17.5±3.5 wt.-%; relative to the total weight of the dosage form and based upon the total weight of all diluents that are contained in the dosage form;
- optionally, at least one glidant; preferably colloidal silica; more preferably with an content within the range of 0.4±0.2 wt.-%; relative to the total weight of the dosage form and based upon the total weight of all glidants that are contained in the dosage form; and
- optionally, at least one lubricant; preferably magnesium stearate; more preferably with an content within the range of 0.3±0.2 wt.-%; relative to the total weight of the dosage form and based upon the total weight of all lubricants that are contained in the dosage form.

In preferred embodiments, the second layer extragranular phase comprises no cellulose ether and/or acrylic polymer; preferably no modified release matrix material.

In other preferred embodiments, the second layer extragranular phase comprises a modified release matrix material comprising or essentially consisting of the first cellulose ether.

In still other preferred embodiments, the second layer extragranular phase comprises a modified release matrix material comprising or essentially consisting of the first cellulose ether and the acrylic polymer.

In still other preferred embodiments, the second layer intragranular phase comprises a modified release matrix material comprising or essentially consisting of the first cellulose ether, the second cellulose ether and the acrylic polymer.

Preferably, the second layer extragranular phase of the bilayer tablet according to the invention comprises or essentially consists of
- a modified release matrix material; preferably comprising or essentially consisting of the first cellulose ether and the acrylic polymer; more preferably with an content within the range of 7.0±3.0 wt.-%; relative to the total weight of the dosage form and based upon the total weight of all modified release matrix material that is contained in the dosage form;
- at least one diluent; preferably microcrystalline cellulose; preferably the first type microcrystalline cellulose and/or the second type microcrystalline cellulose; more preferably with an content within the range of 7.5±3.5 wt.-%; relative to the total weight of the dosage form and based upon the total weight of all diluents that are contained in the dosage form;
- optionally, at least one glidant; preferably colloidal silica; more preferably with an content within the range of 0.4±0.2 wt.-%; relative to the total weight of the dosage form and based upon the total weight of all glidants that are contained in the dosage form; and
- optionally, at least one lubricant; preferably magnesium stearate; more preferably with an content within the range of 0.4±0.2 wt.-%; relative to the total weight of the dosage form and based upon the total weight of all lubricants that are contained in the dosage form.

The excipients of the pharmaceutical dosage form according to the invention may be split into fractions, where a first fraction is contained in the first layer and a second fraction is contained in the second layer. The fractions of excipients may further be split into parts, where a first part of a fraction is contained in the intragranular phase of the respective layer and a second part of this fraction is contained in the extragranular phase of the respective layer. For the purpose of the specification, more than one representative of a particular excipient may be contained in the oral pharmaceutical dosage forms. When these excipients are contained in one and the same phase of a layer of the dosage form, they preferably differ from one another. However, when these excipients are contained in different phases of the layers of the oral dosage form, they may differ from one another or may be the same. For example, the second layer intragranular phase may contain microcrystalline cellulose as diluent in combination with crystalline lactose or mannitol as diluent. Likewise, for example, the first layer intragranular phase, the second layer intragranular phase, and the second layer extragranular phase may all contain colloidal silica as glidant and magnesium stearate as lubricant.

Preferably, the first layer intragranular phase is prepared by dry granulation; preferably roller compaction.

Preferably, the second layer intragranular phase is prepared by dry granulation or wet granulation; preferably dry granulation; more preferably roller compaction.

Another aspect of the invention relates to the dosage form according to the invention for use in the treatment or prevention of a condition or disease selected from the group consisting of hypertension, heart failure, such as (acute and chronic) congestive heart failure, left ventricular dysfunction and hypertrophic cardiomyopathy, diabetic cardiac myopathy, supra-ventricular and ventricular arrhythmias, atrial fibrillation, atrial flutter, detrimental vascular re-modeling, myocardial infarction and its sequelae, atherosclerosis, angina (whether unstable or stable), renal insufficiency (diabetic and non-diabetic), angina pectoris, diabetes, secondary aldosteronism, primary and secondary pulmonary hypertension, renal failure conditions, such as diabetic nephropathy, glomerulonephritis, scleroderma, glomerular sclerosis, proteinuria of primary renal disease, and also renal vascular hypertension, diabetic retinopathy, the management of other vascular disorders, such as migraine, peripheral vascular disease, Raynaud's disease, luminal hyperplasia, cognitive dysfunction, such as Alzheimer's, glaucoma and stroke; preferably hypertension and heart failure.

Another aspect of the invention relates to a process for the preparation of a pharmaceutical dosage form according to the invention, preferably a bilayer tablet, more preferably a bilayer tablet comprising a first layer containing valsartan or the physiologically acceptable salt thereof and a second layer containing indapamide or the physiologically acceptable salt thereof.

In preferred embodiments, the process according to the invention comprises the steps of:
(a) granulating valsartan with one or more excipients; preferably at least one diluent, at least one disintegrant, optionally at least one glidant, and optionally at least one lubricant; thereby obtaining a valsartan granulate; optionally sieving the valsartan granulate;
(b) mixing the valsartan granulate obtained in step (a) with one or more extragranular excipients; preferably at least one diluent, and optionally at least one lubricant; thereby obtaining a valsartan compression mixture;
(c) granulating indapamide with a modified release matrix material and one or more excipients; preferably at least one diluent, optionally at least one glidant, and optionally at least one lubricant; thereby obtaining an indapamide granulate; optionally sieving the indapamide granulate;
(d) mixing the indapamide granulate obtained in step (c) with a modified release matrix material and one or more extragranular excipients; preferably at least one diluent, optionally at least one glidant, and optionally at least one lubricant; thereby obtaining an indapamide compression mixture; and
(e) optionally, compressing the valsartan compression mixture obtained in step (b) and the indapamide compression mixture obtained in step (d) by means of a tablet press thereby obtaining a tablet; preferably a bilayer tablet; more preferably a bilayer tablet comprising a first layer containing valsartan or the physiologically acceptable salt thereof and a second layer containing indapamide or the physiologically acceptable salt thereof;
preferably wherein the modified release matrix material is as defined above, said at least one diluent is as defined above, said at least one disintegrant is as defined above, said optionally at least one glidant is as defined above, and said optionally at least one lubricant is as defined above.

In step (a) of the process according to the invention, valsartan is granulated with one or more excipients thereby obtaining a valsartan granulate.

Preferably, valsartan is dry granulated; more preferably granulation is performed by roller compaction.

In step (c) of the process according to the invention, indapamide is granulated with one or more excipients thereby obtaining an indapamide granulate.

In preferred embodiments, indapamide is dry granulated; more preferably granulation is performed by roller compaction.

In other preferred embodiments, indapamide is wet granulated; preferably granulation is performed in a high-shear mixer.

In these preferred embodiments, step (c) of the process according to the invention comprises the sub-steps of:
(c₁) providing a granulation liquid comprising
   - one or more solvents; preferably water;
   - optionally, one or more excipients;
(c₂) providing a granulation mixture comprising indapamide, a modified release matrix material and one or more excipients; preferably at least one diluent, and optionally at least one glidant;
(c₃) wet granulating the granulation mixture provided in step (c₂) with the granulation liquid provided in step (c₁) thereby obtaining an indapamide granulate; and
(c₄) optionally, drying the indapamide granulate obtained in step (c₃); preferably by means of a fluid bed dryer;
preferably wherein the modified release matrix material is as defined above, said at least one diluent is as defined above, and said optionally at least one glidant is as defined above.

Another aspect of the invention relates to a pharmaceutical dosage form obtainable by the process according to the invention.

Particularly preferred embodiments of the invention are compiled as clauses 1 to 84 hereinafter:
Clause 1: An oral pharmaceutical dosage form, preferably a bilayer tablet comprising a first layer and a second layer, comprising valsartan or a physiologically acceptable salt thereof; preferably wherein the pharmaceutical dosage form provides conventional release of valsartan or the physiologically acceptable salt thereof; and indapamide or a physiologically acceptable salt thereof; preferably wherein the pharmaceutical dosage form provides modified release of indapamide or the physiologically acceptable salt thereof.
Clause 2: The pharmaceutical dosage form according to clause 1, which provides conventional release of valsartan or the physiologically acceptable salt thereof.
Clause 3: The pharmaceutical dosage form according to clause 1 or 2, which provides an *in vitro* dissolution profile using USP I method (Apparatus 1 or Basket apparatus) with baskets 40 mesh at 100 rpm in 900 mL phosphate buffer at pH 6.8 and 37.0°C±0.5°C such that after 15 minutes at least 50 wt.-% of the valsartan that was initially contained in the pharmaceutical dosage form have been released; preferably at least 55 wt.-%, more preferably at least 60 wt.-%, still more preferably at least 65 wt.-%, yet more preferably at least 70 wt.-%, even more preferably at least 75 wt.-%, most preferably at least 80 wt.-%, and in particular at least 85 wt.-%.
Clause 4: The pharmaceutical dosage form according to any of the preceding clauses, which provides modified release of indapamide or the physiologically acceptable salt thereof; preferably prolonged release (retarded release, sustained release), delayed release, or extended release; more preferably prolonged release.
Clause 5: The pharmaceutical dosage form according to any of the preceding clauses, which provides an *in vitro* dissolution profile using USP II method (Apparatus 2 or paddles) at 100 rpm (tablets in Japanese sinkers (JS)) in 900 mL phosphate buffer at pH 6.8 and 37.0°C±0.5°C such that after 2 hours at most 70 wt.-% of the indapamide that was initially contained in the pharmaceutical dosage form have been released; preferably at most 65 wt.-%, more preferably at most 60 wt.-%, still more preferably at most 55 wt.-%, yet more preferably at most 50 wt.-%, even more preferably at most 45 wt.-%, most preferably at most 40 wt.-%, and in particular at most 35 wt.-%.
Clause 6: The pharmaceutical dosage form according to any of the preceding clauses, wherein indapamide is embedded in a modified release matrix comprising a modified release matrix material.
Clause 7: The pharmaceutical dosage form according to clause 6, wherein the modified release matrix comprises or essentially consists of at least one polymer selected from the group consisting of
   (i) cellulose ethers; preferably hydroxypropyl cellulose (HPC), hydroxypropyl methyl cellulose (HPMC), hydroxyethyl cellulose (HEC), methyl cellulose (MC), ethyl cellulose (EC), sodium carboxymethyl cellulose and carboxymethyl cellulose;
   (ii) cellulose esters; preferably cellulose acetate, cellulose acetate succinate, cellulose acetate phthalate, cellulose acetate propionate, cellulose acetate butyrate;
   (iii) acrylic polymers; preferably selected from cross-linked acrylic homopolymers, cross-linked acrylic copolymers, linear acrylic homopolymers, linear acrylic copolymers, cross-linked methacrylic homopolymers, cross-linked methacrylic copolymers, linear methacrylic homopolymers, and linear methacrylic copolymers; preferably selected from poly(acrylic acid), copolymers of acrylic acid, poly(acrylate), acrylate copolymers, poly(methyl acrylate), methyl acrylate copolymers, poly(ethoxyethyl acrylate), ethoxyethyl acrylate copolymers, poly(cyanoethyl acrylate), cyanoethyl acrylate copolymers, poly(aminoalkyl acrylate), aminoalkyl acrylate copolymers, poly(acrylic acid, acrylic acid alkylamide), poly(methacrylic acid, acrylic acid alkylamide), poly(hydroxyethyl acrylate), hydroxyethyl acrylate copolymers, poly(acrylic acid anhydride), acrylic acid anhydride copolymers, poly(acrylamide), acrylamide copolymers, poly(glycidyl acrylate), glycidyl acrylate copolymers, poly(methacrylic acid), methacrylic acid copolymers, poly(methacrylate), methacrylate copolymers, poly(methyl methacrylate), methyl methacrylate copolymers, poly(ethoxyethyl methacrylate), ethoxyethyl methacrylate copolymers, poly(cyanoethyl methacrylate), cyanoethyl methacrylate copolymers, poly(aminoalkyl methacrylate), aminoalkyl methacrylate copolymers, poly(acrylic acid, methacrylic acid alkylamide), poly(methacrylic acid, methacrylic acid alkylamide), poly(hydroxyethyl methacrylate), hydroxyethyl methacrylate copolymers, poly(methacrylic acid anhydride), methacrylic acid anhydride copolymers, poly(methacrylamide), methacrylamide copolymers, poly(glycidyl methacrylate), and glycidyl methacrylate copolymers;
   (iv) polysaccharides or gums; preferably xanthan gum, guar gum, karaya gum, locust bean gum, sodium alginate, carob gum, chitosan, polysaccharides of mannose and galactose, pectin, tragacanth, and agar-agar;
   (v) polyalkylene glycols and polyalkylene oxides;
   (vi) polyvinylalcohol (PVA), cross-linked polyvinylalcohol (PVA), polyvinylpyrrolidone (PVP), cross-linked polyvinylpyrrolidone (PVP), polyvinylpyrrolidone-vinylacetate copolymers, polyvinylchloride (PVC), polyethylene vinyl acetate (PVA), polydimethylsiloxane (PDS), polyether urethane (PEU), polylactic acid (PLA), polyglycolic acid (PGA), polycaprolactone (PCL), polyanhydrides, polyorthoesters; and
   (vii) protein derived materials;
   preferably (i) cellulose ethers and (iii) acrylic polymers.
Clause 8: The pharmaceutical dosage form according to clause 6 or 7, wherein the modified release matrix material comprises or essentially consists of at least one cellulose ether, at least one acrylic polymer, or a mixture of at least one cellulose ether with at least one acrylic polymer.
Clause 9: The pharmaceutical dosage form according to any of clauses 6 to 8, wherein the modified release matrix material comprises or essentially consists of a first cellulose ether, a second cellulose ether, and/or an acrylic polymer.
Clause 10: The pharmaceutical dosage form according to clause 9, wherein the modified release matrix material comprises or essentially consists of the first cellulose ether.
Clause 11: The pharmaceutical dosage form according to any of clauses 6 to 10, wherein the modified release matrix material comprises or essentially consists of two polymers.
Clause 12: The pharmaceutical dosage form according to any of clauses 9 to 11, wherein the modified release matrix material comprises or essentially consists of the first cellulose ether and the second cellulose ether.
Clause 13: The pharmaceutical dosage form according to any of clauses 9 to 11, wherein the modified release matrix material comprises or essentially consists of the first cellulose ether and the acrylic polymer.
Clause 14: The pharmaceutical dosage form according to any of clauses 6 to 13, wherein the modified release matrix material comprises or essentially consists of three polymers.
Clause 15: The pharmaceutical dosage form according to any of clauses 9 to 14, wherein the modified release matrix material comprises or essentially consists of the first cellulose ether, the second cellulose ether and the acrylic polymer.
Clause 16: The pharmaceutical dosage form according to any of clauses 9 to 15, wherein the first cellulose ether and the second cellulose ether are independently of one another selected from hydroxypropyl cellulose (HPC), hydroxypropyl methyl cellulose (HPMC) and hydroxyethyl cellulose (HEC); preferably hydroxypropyl methyl cellulose (HPMC).
Clause 17: The pharmaceutical dosage form according to any of clauses 9 to 16, wherein the first cellulose ether differs from the second cellulose ether; preferably the first cellulose ether and the second cellulose ether are derived from the same monomers but differ in at least one parameter selected from the group consisting of solubility, viscosity and molecular weight.
Clause 18: The pharmaceutical dosage form according to any of clauses 9 to 17, wherein the first cellulose ether has a viscosity at 20°C determined in accordance with ASTM D2363 within the range of from 750 to 8,500 mPa·s, preferably from 1,000 to 8,000 mPa·s, more preferably from 1,250 to 7,500 mPa·s, still more preferably from 1,500 to 7,000 mPa·s, yet more preferably 1,750 to 6,500 mPa·s, even more preferably from 2,000 to 6,000 mPa·s, most preferably from 2,250 to 5,500 mPa·s, and in particular from 2,500 to 5,000 mPa·s.
Clause 19: The pharmaceutical dosage form according to any of clauses 9 to 18, wherein the second cellulose ether has a viscosity at 20°C determined in accordance with ASTM D2363 within the range of from 5,000 to 210,000 mPa·s, preferably from 15,000 to 200,000 mPa·s, more preferably from 25,000 to 190,000 mPa·s, still more preferably from 35,000 to 180,000 mPa·s, yet more preferably 45,000 to 170,000 mPa·s, even more preferably from 55,000 to 160,000 mPa·s, most preferably from 65,000 to 150,000 mPa·s, and in particular from 75,000 to 140,000 mPa·s.
Clause 20: The pharmaceutical dosage form according to any of clauses 7 to 19, wherein the acrylic polymer is a polymer of acrylic acid; preferably a carbomer (poly(acrylic acid), preferably crosslinked). Clause 21: The pharmaceutical dosage form according to any of clauses 6 to 20, wherein the total content of said modified release matrix material is within the range of 21±19 wt.-%, preferably 19±17 wt.-%, more preferably 17±15 wt.-%, still more preferably 15±13 wt.-%, yet more preferably 15±11 wt.-%, even more preferably 15±9.0 wt.-%, most preferably 15±7.0 wt.-%, and in particular 15±5.0 wt.-%; in each case relative to the total weight of the dosage form and based upon the total weight of all modified release matrix material that is contained in the dosage form.
Clause 22: The pharmaceutical dosage form according to any of clauses 6 to 21, wherein the total content of said modified release matrix material is within the range of 37±19 wt.-%, preferably 37±17 wt.-%, more preferably 37±15 wt.-%, still more preferably 37±13 wt.-%, yet more preferably 37±11 wt.-%, even more preferably 37±9.0 wt.-%, most preferably 37±7.0 wt.-%, and in particular 37±5.0 wt.-%; in each case relative to the total weight of the second layer and based upon the total weight of all modified release matrix material that is contained in the second layer.
Clause 23: The pharmaceutical dosage form according to any of clauses 9 to 22, wherein the total content of the first cellulose ether is within the range of 20±18 wt.-%, preferably 17±15 wt.-%, more preferably 13±12 wt.-%, still more preferably 13±10 wt.-%, yet more preferably 13±8.0 wt.-%, even more preferably 13±6.0 wt.-%, most preferably 13±4.0 wt.-%, and in particular 13±2.0 wt.-%; in each case relative to the total weight of the dosage form and based upon the total weight of all first cellulose ether that is contained in the dosage form.
Clause 24: The pharmaceutical dosage form according to any of clauses 9 to 23, wherein the total content of the first cellulose ether is within the range of 32±18 wt.-%, preferably 32±15 wt.-%, more preferably 32±12 wt.-%, still more preferably 32±10 wt.-%, yet more preferably 32±8.0 wt.-%, even more preferably 32±6.0 wt.-%, most preferably 32±4.0 wt.-%, and in particular 32±2.0 wt.-%; in each case relative to the total weight of the second layer and based upon the total weight of all first cellulose ether that is contained in the second layer.
Clause 25: The pharmaceutical dosage form according to any of clauses 9 to 24, wherein the total content of the second cellulose ether is within the range of 10±9.0 wt.-%, preferably 9.0±8.0 wt.-%, more preferably 8.0±7.0 wt.-%, still more preferably 7.0±6.0 wt.-%, yet more preferably 6.0±5.0 wt.-%, even more preferably 6.0±4.0 wt.-%, most preferably 6.0±3.0 wt.-%, and in particular 6.0±2.0 wt.-%; in each case relative to the total weight of the dosage form and based upon the total weight of all second cellulose ether that is contained in the dosage form.
Clause 26: The pharmaceutical dosage form according to any of clauses 9 to 25, wherein the total content of the second cellulose ether is within the range of 10±9.0 wt.-%, preferably 10±8.0 wt.-%, more preferably 10±7.0 wt.-%, still more preferably 10±6.0 wt.-%, yet more preferably 10±5.0 wt.-%, even more preferably 10±4.0 wt.-%, most preferably 10±3.0 wt.-%, and in particular 10±2.0 wt.-%; in each case relative to the total weight of the second layer and based upon the total weight of all second cellulose ether that is contained in the second layer.
Clause 27: The pharmaceutical dosage form according to any of clauses 9 to 26, wherein the total content of the acrylic polymer is within the range of 5.0±4.5 wt.-%, preferably 4.5±4.0 wt.-%, more preferably 4.0±3.5 wt.-%, still more preferably 3.5±3.0 wt.-%, yet more preferably 3.0±2.5 wt.-%, even more preferably 2.5±2.0 wt.-%, most preferably 2.0±1.5 wt.-%, and in particular 2.0±1.0 wt.-%; in each case relative to the total weight of the dosage form and based upon the total weight of all acrylic polymer that is contained in the dosage form.
Clause 28: The pharmaceutical dosage form according to any of clauses 9 to 27, wherein the total content of the acrylic polymer is within the range of 8.0±4.5 wt.-%, preferably 7.5±4.0 wt.-%, more preferably 7.0±3.5 wt.-%, still more preferably 6.5±3.0 wt.-%, yet more preferably 6.0±2.5 wt.-%, even more preferably 5.5±2.0 wt.-%, most preferably 5.0±1.5 wt.-%, and in particular 4.0±1.0 wt.-%; in each case relative to the total weight of the second layer and based upon the total weight of all acrylic polymer that is contained in the second layer.
Clause 29: The pharmaceutical dosage form according to any of the preceding clauses, which comprises one or more excipients independently of one another selected from diluents, disintegrants, glidants, lubricants, coloring agents, and binders.
Clause 30: The pharmaceutical dosage form according to any of the preceding clauses, which comprises at least one disintegrant; preferably selected from the group consisting of crospovidone, starch, maize starch, native starch, pregelatinized starch, sodium starch glycolate, hydroxypropyl starch, microcrystalline cellulose, sodium and/or calcium salts of carboxymethyl cellulose, cross-linked carboxymethylcellulose (e.g. croscarmellose sodium and/or croscarmellose calcium), low substituted hydroxypropyl cellulose, methylcellulose, polacrilin potassium, docusate sodium, alginic acid, sodium and/or calcium alginate, agar, guar gum, chitosan, and combinations thereof; preferably crospovidone.
Clause 31: The pharmaceutical dosage form according to clause 30, wherein the overall content of said at least one disintegrant is within the range of 5.5±5.0 wt.-%, preferably 5.0±4.5 wt.-%, more preferably 4.4±4.0 wt.-%, still more preferably 4.4±3.5 wt.-%, yet more preferably 4.4±3.0 wt.-%, even more preferably 4.4±2.5 wt.-%, most preferably 4.4±2.0 wt.-%, and in particular 4.4±1.5 wt.-%; in each case relative to the total weight of the dosage form and based upon the total weight of all disintegrants that are contained in the dosage form.
Clause 32: The pharmaceutical dosage form according to clause 30 or 31, wherein the overall content of said at least one disintegrant is within the range of 7.4±6.5 wt.-%, preferably 7.4±6.0 wt.-%, more preferably 7.4±5.5 wt.-%, still more preferably 7.4±5.0 wt.-%, yet more preferably 7.4±4.5 wt.-%, even more preferably 7.4±4.0 wt.-%, most preferably 7.4±3.5 wt.-%, and in particular 7.4±3.0 wt.-%; in each case relative to the total weight of the first layer and based upon the total weight of all disintegrants that are contained in the first layer.
Clause 33: The pharmaceutical dosage form according to any of the preceding clauses, which comprises at least one diluent; preferably selected from the group consisting of lactose; preferably lactose monohydrate, anhydrous lactose, amorphous lactose, crystalline lactose, and spray dried and/or granulated lactose; monosaccharides, disaccharides and oligosaccharides; preferably sucrose, glucose, fructose, dextrates, saccharose, raffinose, trehalose, and dextrin, sugar alcohols; preferably xylitol, mannitol, maltitol, isomalt, and sorbitol; cellulose or cellulose derivatives; preferably powdered cellulose, microcrystalline cellulose, and silicified microcrystalline cellulose; starch or starch derivatives; preferably low moisture starch, corn starch, pregelatinized starch, and low moisture pregelatinized starch; and combinations thereof; preferably lactose, sugar alcohols, cellulose and combinations thereof; more preferably crystalline lactose, mannitol, microcrystalline cellulose and combinations thereof; still more preferably mannitol, microcrystalline cellulose and combinations thereof.
Clause 34: The pharmaceutical dosage form according to clause 33, wherein the overall content of said at least one diluent is within the range of 50±47.5 wt.-%, preferably 50±45 wt.-%, more preferably 50±40 wt.-%, still more preferably 50±35 wt.-%, yet more preferably 50±30 wt.-%, even more preferably 50±25 wt.-%, most preferably 50±20 wt.-%, and in particular 50±15 wt.-%; in each case relative to the total weight of the dosage form and based upon the total weight of all diluents that are contained in the dosage form.
Clause 35: The pharmaceutical dosage form according to clause 33 or 34, wherein the overall content of said at least one diluent is within the range of 50±47.5 wt.-%, preferably 50±45 wt.-%, more preferably 50±40 wt.-%, still more preferably 50±35 wt.-%, yet more preferably 50±30 wt.-%, even more preferably 50±25 wt.-%, most preferably 40±20 wt.-%, and in particular 40±17 wt.-%; in each case relative to the total weight of the first layer and based upon the total weight of all diluents that are contained in the first layer.
Clause 36: The pharmaceutical dosage form according to any of clauses 33 to 35, wherein the overall content of said at least one diluent is within the range of 50±47.5 wt.-%, preferably 50±45 wt.-%, more preferably 50±40 wt.-%, still more preferably 50±35 wt.-%, yet more preferably 50±30 wt.-%, even more preferably 50±25 wt.-%, most preferably 50±20 wt.-%, and in particular 50±15 wt.-%; in each case relative to the total weight of the second layer and based upon the total weight of all diluents that are contained in the second layer.
Clause 37: The pharmaceutical dosage form according to any of the preceding clauses, which comprises at least one glidant; preferably selected from the group consisting of colloidal silica, talc, magnesium trisilicate, and combinations thereof; preferably colloidal silica.
Clause 38: The pharmaceutical dosage form according to clause 37, wherein the overall content of said at least one glidant is within the range of 2.5±2.0 wt.-%, preferably 2.0±1.75 wt.-%, more preferably 1.75±1.5 wt.-%, still more preferably 1.5±1.25 wt.-%, yet more preferably 1.25±1.0 wt.-%, even more preferably 1.25±0.75 wt.-%, most preferably 1.25±0.5 wt.-%, and in particular 1.25±0.25 wt.-%; in each case relative to the total weight of the dosage form and based upon the total weight of all glidants that are contained in the dosage form.
Clause 39: The pharmaceutical dosage form according to clause 37 or 38, wherein the overall content of said at least one glidant is within the range of 2.5±2.0 wt.-%, preferably 2.0±1.75 wt.-%, more preferably 1.75±1.5 wt.-%, still more preferably 1.5±1.25 wt.-%, yet more preferably 1.5±1.0 wt.-%, even more preferably 1.5±0.75 wt.-%, and most preferably 1.5±0.5 wt.-%, in each case relative to the total weight of the first layer and based upon the total weight of all glidants that are contained in the first layer.
Clause 40: The pharmaceutical dosage form according to any of clauses 37 to 39, wherein the overall content of said at least one glidant is within the range of 2.5±2.0 wt.-%, preferably 2.0±1.75 wt.-%, more preferably 1.75±1.5 wt.-%, still more preferably 1.5±1.25 wt.-%, yet more preferably 1.5±1.0 wt.-%, even more preferably 1.5±0.75 wt.-%, most preferably 1.5±0.5 wt.-%, and in particular 1.5±0.25 wt.-%; in each case relative to the total weight of the second layer and based upon the total weight of all glidants that are contained in the second layer.
Clause 41: The pharmaceutical dosage form according to any of the preceding clauses, which comprises at least one lubricant; preferably selected from stearic acid, magnesium stearate, calcium stearate, aluminum stearate, zinc stearate, magnesium palmitate, magnesium oleate, sodium stearyl fumarate, glycerol tribehenate and combinations thereof; preferably magnesium stearate.
Clause 42: The pharmaceutical dosage form according to clause 41, wherein the overall content of said at least one lubricant is within the range of 2.0±1.8 wt.-%, preferably 1.8±1.6 wt.-%, more preferably 1.8±1.4 wt.-%, still more preferably 1.8±1.2 wt.-%, yet more preferably 1.8±1.0 wt.-%, even more preferably 1.8±0.8 wt.-%, most preferably 1.8±0.6 wt.-%, and in particular 1.8±0.4 wt.-%; in each case relative to the total weight of the dosage form and based upon the total weight of all lubricants that are contained in the dosage form.
Clause 43: The pharmaceutical dosage form according to clause 41 or 42, wherein the overall content of said at least one lubricant is within the range of 2.0±1.8 wt.-%, preferably 2.0±1.6 wt.-%, more preferably 2.0±1.4 wt.-%, still more preferably 2.0±1.2 wt.-%, yet more preferably 2.0±1.0 wt.-%, even more preferably 2.0±0.8 wt.-%, most preferably 2.0±0.6 wt.-%, and in particular 2.0±0.5 wt.-%; in each case relative to the total weight of the first layer and based upon the total weight of all lubricants that are contained in the first layer.
Clause 44: The pharmaceutical dosage form according to any of clauses 41 to 43, wherein the overall content of said at least one lubricant is within the range of 2.0±1.8 wt.-%, preferably 1.8±1.6 wt.-%, more preferably 1.8±1.4 wt.-%, still more preferably 1.8±1.2 wt.-%, yet more preferably 1.8±1.0 wt.-%, even more preferably 1.0±0.8 wt.-%, most preferably 1.0±0.6 wt.-%, and in particular 1.0±0.4 wt.-%; in each case relative to the total weight of the second layer and based upon the total weight of all lubricants that are contained in the second layer.
Clause 45: The pharmaceutical dosage form according to any of the preceding clauses, wherein the content of valsartan or the physiologically acceptable salt thereof is within the range of 24±22 wt.-%, preferably 24±20 wt.-%, more preferably 24±18 wt.-%, still more preferably 24±16 wt.-%, yet more preferably 24±14 wt.-%, even more preferably 24±12 wt.-%, most preferably 24±10 wt.-%, and in particular 24±8.0 wt.-%; in each case relative to the total weight of the dosage form and expressed as equivalent weight relative to the non-salt form of valsartan.
Clause 46: The pharmaceutical dosage form according to any of the preceding clauses, wherein the content of valsartan or the physiologically acceptable salt thereof is within the range of 40±28 wt.-%, preferably 40±26 wt.-%, more preferably 40±24 wt.-%, still more preferably 40±22 wt.-%, yet more preferably 40±20 wt.-%, even more preferably 40±18 wt.-%, most preferably 40±16 wt.-%, and in particular 40±14 wt.-%; in each case relative to the total weight of the first layer and expressed as equivalent weight relative to the non-salt form of valsartan.
Clause 47: The pharmaceutical dosage form according to any of the preceding clauses, wherein the content of indapamide or the physiologically acceptable salt thereof is within the range of 0.3±0.275 wt.-%, preferably 0.3±0.2 wt.-%, more preferably 0.3±0.175 wt.-%, still more preferably 0.3±0.15 wt.-%, yet more preferably 0.3±0.125 wt.-%, even more preferably 0.3±0.1 wt.-%, most preferably 0.3±0.075 wt.-%, and in particular 0.3±0.05 wt.-%; in each case relative to the total weight of the dosage form and expressed as equivalent weight relative to the non-salt form of indapamide.
Clause 48: The pharmaceutical dosage form according to any of the preceding clauses, wherein the content of indapamide or the physiologically acceptable salt thereof is within the range of 0.75±0.275 wt.-%, preferably 0.75±0.2 wt.-%, more preferably 0.75±0.175 wt.-%, still more preferably 0.75±0.15 wt.-%, yet more preferably 0.75±0.125 wt.-%, even more preferably 0.75±0.1 wt.-%, most preferably 0.75±0.075 wt.-%, and in particular 0.75±0.05 wt.-%; in each case relative to the total weight of the second layer and expressed as equivalent weight relative to the non-salt form of indapamide.
Clause 49: The pharmaceutical dosage form according to any of the preceding clauses, wherein the valsartan is present in the non-salt form of valsartan.
Clause 50: The pharmaceutical dosage form according to any of the preceding clauses, wherein the indapamide is present in the non-salt form of indapamide.
Clause 51: The pharmaceutical dosage form according to any of the preceding clauses, which does not contain any other pharmaceutically active ingredients besides valsartan or the physiologically acceptable salt thereof and indapamide or the physiologically acceptable salt thereof.
Clause 52: The pharmaceutical dosage form according to any of the preceding clauses, which is selected from the group consisting of tablets, pills, powders, lozenges, sachets, soft gelatin capsules, hard gelatin capsules, and suppositories; preferably tablets; more preferably bilayer tablets.
Clause 53: The pharmaceutical dosage form according to any of the preceding clauses, which is coated; preferably with a water-soluble coating material.
Clause 54: The pharmaceutical dosage form according to any of the preceding clauses, which has a diameter of at least 3.0 mm, preferably at least 4.0 mm, more preferably at least 5.0 mm, still more preferably at least 6.0 mm, yet more preferably at least 7.0 mm, even more preferably at least 8.0 mm, most preferably at least 9.0 mm, and in particular at least 10 mm.
Clause 55: The pharmaceutical dosage form according to any of the preceding clauses, which has a diameter of at most 17 mm, preferably at most 16 mm, more preferably at most 15 mm, still more preferably at most 14 mm, yet more preferably at most 13 mm, even more preferably at most 12 mm. Clause 56: The pharmaceutical dosage form according to any of the preceding clauses, which has a diameter within the range of 10±6.0 mm, preferably 10±4.0 mm, more preferably 10±2.0 mm.
Clause 57: The pharmaceutical dosage form according to any of the preceding clauses, which has a thickness within the range of 5.0±4.0 mm, preferably 5.0±3.5 mm, more preferably 5.0±3.0 mm, still more preferably 5.0±2.5 mm, yet more preferably 5.0±2.0 mm, even more preferably 5.0±1.5 mm, most preferably 5.0±1.0 mm, and in particular 5.0±0.7 mm.
Clause 58: The pharmaceutical dosage form according to any of the preceding clauses, which comprises a first unit (preferably a first layer) that contains essentially the total amount of valsartan or the physiologically acceptable salt thereof; and a second unit (preferably a second layer) that is spatially separated from the first unit and that contains essentially the total amount of indapamide or the physiologically acceptable salt thereof.
Clause 59: The pharmaceutical dosage form according to clause 58, wherein the relative weight ratio of the first unit (preferably the first layer) to the second unit (preferably the second layer) is from 4:1 to 1:1, preferably from 3:1 to 1:1, more preferably from 2:1 to 1:1.
Clause 60: The pharmaceutical dosage form according to any of the preceding clauses, wherein the dosage form is a bilayer tablet comprising a first layer which comprises valsartan or the physiologically acceptable salt thereof and a second layer which comprises indapamide or the physiologically acceptable salt thereof.
Clause 61: The pharmaceutical dosage form according to clause 60, wherein the first layer comprises essentially the total amount of valsartan or the physiologically acceptable salt thereof that is contained in the pharmaceutical dosage form.
Clause 62: The pharmaceutical dosage form according to clause 60 or 61, wherein the second layer comprises essentially the total amount of indapamide or the physiologically acceptable salt thereof that is contained in the pharmaceutical dosage form.
Clause 63: The pharmaceutical dosage form according to any of clauses 60 to 62, wherein the first layer comprises a first layer intragranular phase and a first layer extragranular phase.
Clause 64: The pharmaceutical dosage form according to any of clauses 60 to 63, wherein valsartan or the physiologically acceptable salt thereof is contained in the first layer intragranular phase.
Clause 65: The pharmaceutical dosage form according to any of clauses 60 to 64, wherein the first layer, preferably the first layer intragranular phase, comprises at least one disintegrant as defined in any of clauses 30 to 32.
Clause 66: The pharmaceutical dosage form according to any of clauses 60 to 65, wherein the first layer, preferably the first layer intragranular phase and the first layer extragranular phase, comprises at least one diluent as defined in any of clauses 33 to 36; preferably microcrystalline cellulose.
Clause 67: The pharmaceutical dosage form according to any of clauses 60 to 66, wherein the first layer, preferably the first layer intragranular phase, comprises at least one glidant as defined in any of clauses 37 to 40.
Clause 68: The pharmaceutical dosage form according to any of clauses 60 to 67, wherein the first layer, preferably the first layer intragranular phase and the first layer extragranular phase, comprises at least one lubricant as defined in any of clauses 41 to 44.
Clause 69: The pharmaceutical dosage form according to any of clauses 60 to 68, wherein the second layer comprises a second layer intragranular phase and a second layer extragranular phase.
Clause 70: The pharmaceutical dosage form according to any of clauses 60 to 69, wherein indapamide or the physiologically acceptable salt thereof is contained in the second layer intragranular phase. Clause 71: The pharmaceutical dosage form according to any of clauses 60 to 70, wherein the second layer, preferably the second layer intragranular phase and the second layer extragranular phase, comprises a modified release matrix material as defined in any of clauses 6 to 28.
Clause 72: The pharmaceutical dosage form according to any of clauses 60 to 71, wherein the second layer, preferably the second layer intragranular phase and the second layer extragranular phase, comprises at least one diluent as defined in any of clauses 33 to 36; preferably microcrystalline cellulose and crystalline lactose, or microcrystalline cellulose and mannitol; more preferably microcrystalline cellulose and mannitol.
Clause 73: The pharmaceutical dosage form according to any of clauses 60 to 72, wherein the second layer, preferably the second layer intragranular phase and the second layer extragranular phase, comprises at least one glidant as defined in any of clauses 37 to 40.
Clause 74: The pharmaceutical dosage form according to any of clauses 60 to 73, wherein the second layer, preferably the second layer intragranular phase and the second layer extragranular phase, comprises at least one lubricant as defined in any of clauses 41 to 44.
Clause 75: The pharmaceutical dosage form according to any of clauses 60 to 74, wherein the first layer intragranular phase is prepared by dry granulation; preferably roller compaction.
Clause 76: The pharmaceutical dosage form according to any of clauses 60 to 75, wherein the second layer intragranular phase is prepared by dry granulation or wet granulation; preferably dry granulation; more preferably roller compaction.
Clause 77: The pharmaceutical dosage form according to any of the preceding clauses for use in the treatment or prevention of a condition or disease selected from the group consisting of hypertension, heart failure, such as (acute and chronic) congestive heart failure, left ventricular dysfunction and hypertrophic cardiomyopathy, diabetic cardiac myopathy, supra-ventricular and ventricular arrhythmias, atrial fibrillation, atrial flutter, detrimental vascular re-modeling, myocardial infarction and its sequelae, atherosclerosis, angina (whether unstable or stable), renal insufficiency (diabetic and non-diabetic), angina pectoris, diabetes, secondary aldosteronism, primary and secondary pulmonary hypertension, renal failure conditions, such as diabetic nephropathy, glomerulonephritis, scleroderma, glomerular sclerosis, proteinuria of primary renal disease, and also renal vascular hypertension, diabetic retinopathy, the management of other vascular disorders, such as migraine, peripheral vascular disease, Raynaud's disease, luminal hyperplasia, cognitive dysfunction, such as Alzheimer's, glaucoma and stroke; preferably hypertension and heart failure.
Clause 78: A process for the preparation of a pharmaceutical dosage form according to any of the preceding clauses.
Clause 79: The process according to clause 78, which comprises the steps of: granulating valsartan with one or more excipients; preferably at least one diluent, at least one disintegrant, optionally at least one glidant, and optionally at least one lubricant; thereby obtaining a valsartan granulate; mixing the valsartan granulate obtained in step (a) with one or more extragranular excipients; preferably at least one diluent, and optionally at least one lubricant; thereby obtaining a valsartan compression mixture; optionally sieving the valsartan granulate; granulating indapamide with a modified release matrix material and one or more excipients; preferably at least one diluent, optionally at least one glidant, and optionally at least one lubricant; thereby obtaining an indapamide granulate; optionally sieving the indapamide granulate; mixing the indapamide granulate obtained in step (c) with a modified release matrix material and one or more extragranular excipients; preferably at least one diluent, optionally at least one glidant, and optionally at least one lubricant; thereby obtaining an indapamide compression mixture; and optionally, compressing the valsartan compression mixture obtained in step (b) and the indapamide compression mixture obtained in step (d) by means of a tablet press thereby obtaining a tablet; preferably a bilayer tablet; more preferably a bilayer tablet comprising a first layer containing valsartan or the physiologically acceptable salt thereof and a second layer containing indapamide or the physiologically acceptable salt thereof; preferably wherein the modified release matrix material is as defined in any of clauses 6 to 28, said at least one diluent is as defined in any of clauses 33 to 36, said at least one disintegrant is as defined in any of clauses 30 to 32, said optionally at least one glidant is as defined in any of clauses 37 to 40, and said optionally at least one lubricant is as defined in any of clauses 41 to 44. Clause 80: The process according to clause 78 or 79, wherein valsartan is dry granulated; preferably granulation is performed by roller compaction.
Clause 81: The process according to any of clauses 78 to 80, wherein indapamide is dry granulated; more preferably granulation is performed by roller compaction.
Clause 82: The process according to any of clauses 78 to 81, wherein indapamide is wet granulated; preferably granulation is performed in a high-shear mixer.
Clause 83: The process according to any of clauses 79 to 82, wherein step (c) comprises the sub-steps of: (c₁) providing a granulation liquid comprising one or more solvents; preferably water; optionally, one or more excipients; (c₂) providing a granulation mixture comprising indapamide, a modified release matrix material and one or more excipients; preferably at least one diluent, and optionally at least one glidant; (c₃) wet granulating the granulation mixture provided in step (c₂) with the granulation liquid provided in step (c₁) thereby obtaining an indapamide granulate; and (f) optionally, drying the indapamide granulate; obtained in step (c₃); preferably by means of a fluid bed dryer; preferably wherein the modified release matrix material is as defined in any of clauses 6 to 28, said at least one diluent is as defined in any of clauses 33 to 36, and said optionally at least one glidant is as defined in any of clauses 37 to 40.
Clause 84: A pharmaceutical dosage form obtainable by the process according to any of clauses 78 to 83.

The following examples further illustrate the invention but are not to be construed as limiting its scope.

Bilayer tablets were prepared from two formulations, a first formulation for the first layer and a second formulation for the second layer.

Examples V-1 to V-8 relate to formulations for the first layer comprising valsartan, whereas Examples I-1 to I-13 relate to formulations for the second layer comprising indapamide.

Examples 1 to 14 relate to the preparation of bilayer tablets combining specific formulations selected from Examples V-1 to V-8 with specific formulations selected from Examples I-1 to 1-13.

### Examples V-1 to V-8 - Preparation of formulation for first layer comprising valsartan:

| VALSARTAN LAYER [mg] | | Examples | | | | | | | |
|---|---|---|---|---|---|---|---|---|---|
| | | V-1 | V-2 | V-3 | V-4 | V-5 | V-6 | V-7 | V-8 |
| *Intragranular excipients* | | | | | | | | | |
| active ingredient | Valsartan | 80.00 | 80.00 | 160.00 | 160.00 | 160.00 | 80.00 | 80.00 | 80.00 |
| diluent | Microcrystalline cellulose, Type 112/Type 102 | - | - | 62.70 | - | - | 161.85 | 142.70 | 107.85 |
| diluent | Microcrystalline cellulose, Type 200 LM/Type 200 | 31.50 | 32.85 | - | 62.70 | 76.70 | - | - | 54.00 |
| disintegrant | Crospovidone (Kollidon CL^{®}) | 15.00 | 14.50 | 29.00 | 29.00 | 15.00 | 14.50 | 29.00 | 14.50 |
| glidant | Colloidal silica | 1.50 | 1.50 | 4.50 | 4.50 | 4.50 | 2.25 | 4.50 | 2.25 |
| lubricant | Magnesium stearate | 2.50 | 1.65 | 4.80 | 4.80 | 4.80 | 2.40 | 4.80 | 2.40 |
| *Extragranular excipients* | | | | | | | | | |
| diluent | Microcrystalline cellulose, Type 112/Type 102 | 17.23 | 17.88 | 30.00 | 36.30 | 36.30 | 36.30 | 36.30 | 36.30 |
| diluent | Microcrystalline cellulose, Type 101 | - | - | 5.76 | - | - | - | - | - |
| lubricant | Magnesium stearate | 2.00 | 1.35 | 2.70 | 2.70 | 2.70 | 2.70 | 2.70 | 2.70 |
| coloring agent | Iron oxide, Yellow | 0.27 | 0.27 | 0.54 | - | - | - | - | - |
| *Mass of Valsartan layer* | | *150.00* | *150.00* | *300.00* | *300.00* | *300.00* | *300.00* | *300.00* | *300.00* |

Valsartan, part of microcrystalline cellulose, crospovidone, colloidal silica and part of magnesium stearate were granulated with a roller compactor and sieved. The remaining part of microcrystalline cellulose, optionally colorant(s) ferric oxide(s), if present in formulation, and magnesium stearate were added to the sieved granulate and mixed in a stainless steel container to obtain valsartan compression mixture.

### Examples 1-1 to 1-7 - Preparation of formulation for second layer comprising indapamide:

| INDAPAMIDE LAYER [mg] | | Examples | | | | | | |
|---|---|---|---|---|---|---|---|---|
| | | I-1 | I-2 | I-3 | I-4 | I-5 | I-6 | I-7 |
| *Intragranular excipients* | | | | | | | | |
| active ingredient | Indapamide | 1.50 | 1.50 | 1.50 | 1.50 | 1.50 | 1.50 | 1.50 |
| modified release matrix material | Hypromellose K4M | 60.00 | 80.00 | 80.00 | 44.00 | 60.00 | 44.00 | 44.00 |
| modified release matrix material | Hypromellose K100M | - | 20.00 | 20.00 | - | - | - | - |
| diluent | Microcrystalline cellulose, Type 101 | 29.30 | 28.80 | 28.80 | 36.00 | 20.00 | 36.00 | 36.00 |
| diluent | Lactose crystalline 200 mesh | 34.30 | 33.70 | 33.70 | 41.00 | 41.00 | 41.00 | 41.00 |
| glidant | Colloidal silica | 2.00 | 2.00 | 2.00 | 2.00 | 2.00 | 2.00 | 2.00 |
| granulating liquid | Purified water | q.s. | q.s. | q.s. | q.s. | q.s. | q.s. | q.s. |
| *Extragranular excipients* | | | | | | | | |
| modified release matrix material | Hypromellose K4M | 39.40 | - | - | 20.00 | 40.00 | 20.00 | 20.00 |
| modified release matrix material | Hypromellose K100M | - | - | - | - | - | 20.00 | - |
| modified release matrix material | Carbomer | - | - | - | 5.00 | 10.00 | 10.00 | 10.00 |
| diluent | Microcrystalline cellulose, Type 112/Type 102 | 30.50 | 31.00 | 31.00 | 47.50 | 19.20 | 19.20 | 39.20 |
| diluent | Microcrystalline cellulose, Type 101 | - | - | - | - | 3.00 | 3.00 | 3.00 |
| glidant | Colloidal silica | 1.00 | 1.00 | 1.00 | 1.00 | 1.00 | 1.00 | 1.00 |
| lubricant | Magnesium stearate | 2.00 | 2.00 | 2.00 | 2.00 | 2.00 | 2.00 | 2.00 |
| coloring agent | Iron oxide, Yellow | - | - | - | - | 0.30 | 0.30 | 0.30 |
| *Mass of indapamide layer* | | *200.00* | *200.00* | *200.00* | *200.00* | *200.00* | *200.00* | *200.00* |

Indapamide, part of hypromellose, part of microcrystalline cellulose, lactose and part of colloidal silica were granulated with water in a high-shear mixer. Wet granulate was dried in fluid bed dryer. The remaining part of hypromellose, the remaining part of microcrystalline cellulose, the remaining part of colloidal silica, carbomer, if present in formulation, optionally colorant(s) ferric oxide(s) and magnesium stearate were added to the dry granulate and mixed in a stainless steel container to obtain indapamide compression mixture.

### Examples I-8 to I-13 - Preparation of formulation for second layer comprising indapamide:

| INDAPAMIDE LAYER [mg] | | Examples | | | | | |
|---|---|---|---|---|---|---|---|
| | | I-8 | I-9 | I-10 | I-11 | I-12 | I-13 |
| *Intragranular excipients* | | | | | | | |
| active ingredient | Indapamide | 1.50 | 1.50 | 1.50 | 1.50 | 1.50 | 1.50 |
| modified release matrix material | Hypromellose K4M | 44.00 | 44.00 | 44.00 | 44.00 | 44.00 | 44.00 |
| diluent | Microcrystalline cellulose, Type 112/Type 102 | 35.00 | 63.50 | 50.50 | 35.50 | 50.50 | 35.50 |
| diluent | Microcrystalline cellulose, Type 101 | - | - | - | 15.00 | - | 15.00 |
| diluent | Lactose crystalline 200 mesh | 41.00 | 41.00 | - | - | - | - |
| diluent | Mannitol, Type 200 | - | - | 41.00 | 20.00 | 41.00 | 20.00 |
| diluent | Mannitol, Type 160C | - | - | - | 21.00 | - | 21.00 |
| glidant | Colloidal silica | 2.00 | 2.00 | 2.00 | 2.00 | 2.00 | 2.00 |
| lubricant | Magnesium stearate | 1.00 | 1.00 | 1.00 | 1.00 | 1.00 | 1.00 |
| *Extragranular excipients* | | | | | | | |
| modified release matrix material | Hypromellose K4M | 20.00 | 20.00 | 20.00 | 20.00 | 20.00 | 20.00 |
| modified release matrix material | Hypromellose K100M | - | - | - | - | - | - |
| modified release matrix material | Carbomer | - | 5.00 | 10.00 | 10.00 | 10.00 | 10.00 |
| diluent | Microcrystalline cellulose, Type 112/Type 102 | 53.50 | 20.00 | 24.70 | 24.70 | 25.80 | 25.80 |
| diluent | Microcrystalline cellulose, Type 101 | - | - | 3.00 | 3.00 | 2.00 | 2.00 |
| glidant | Colloidal silica | 1.00 | 1.00 | 1.00 | 1.00 | 1.00 | 1.00 |
| lubricant | Magnesium stearate | 1.00 | 1.00 | 1.00 | 1.00 | 1.00 | 1.00 |
| coloring agent | Iron oxide, Yellow or Iron oxide, Red | - | - | 0.30 | 0.30 | 0.20 | 0.20 |
| *Mass of Indapamide layer* | | *200.00* | *200.00* | *200.00* | *200.00* | *200.00* | *200.00* |

Indapamide, part of hypromellose, part of microcrystalline cellulose, lactose, mannitol, part of colloidal silica and part of magnesium stearate were granulated with a roller compactor and sieved. The remaining part of hypromellose, the remaining part of microcrystalline cellulose, the remaining part of colloidal silica, carbomer, if present in formulation, optionally colorant(s) ferric oxide(s) and magnesium stearate were added to the sieved granulate and mixed in a stainless steel container to obtain indapamide compression mixture.

The particle size of three different types of mannitol (Parteck^{®} M200, Pearlitol^{®} 200 SD, and Pearlitol^{®} 160C) was measured in accordance with USP <429> "*Laser Diffraction Measurement of Particle Size*" (n=1)) and the results are compiled in the table here below:

| [µM] | Dv10 | Dv50c | Dv90c | CEDc(vol) |
|---|---|---|---|---|
| Parteck^{®} M200 | 73.0 | 162 | 343 | 190 |
| Pearlitol^{®} 200 SD | 95.0 | 153 | 225 | 157 |
| Pearlitol^{®} 160C | 69 | 170 | 328 | 188 |

| | | | | |
|---|---|---|---|---|
| CED = circular equivalent diameter (area equivalent diameter) | | | | |

### Examples 1 to 14 - preparation of bilayer tablets:

Indapamide compression mixture and valsartan compression mixture were compressed to bilayer tablets using bilayer tableting machine.

The following bilayer tablets were prepared:

| | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| First layer (valsartan) | V-1 | V-2 | V-2 | V-3 | V-3 | V-3 | V-4 | V-4 | V-4 | V-4 | V-5 | V-4 | V-6 | V-7 |
| Second layer (indapamide) | I-1 | I-2 | I-3 | I-8 | I-9 | I-4 | I-6 | I-10 | I-7 | I-11 | I-11 | I-12 | I-12 | I-13 |
| Tablet weight | 350 | 350 | 350 | 500 | 500 | 500 | 500 | 500 | 500 | 500 | 500 | 500 | 500 | 500 |
| Tablet diameter [mm] | 10 | 10 | Oval, 13x8 | 11 | 11 | 11 | 11 | 11 | 11 | 11 | 11 | 11 | 11 | 11 |

## Claims

1. An oral pharmaceutical dosage form, preferably a bilayer tablet comprising a first layer and a second layer, the pharmaceutical dosage form comprising
(i) valsartan or a physiologically acceptable salt thereof; preferably wherein the pharmaceutical dosage form provides conventional release of valsartan or the physiologically acceptable salt thereof; and
(ii) indapamide or a physiologically acceptable salt thereof; preferably wherein the pharmaceutical dosage form provides modified release of indapamide or the physiologically acceptable salt thereof.

2. The pharmaceutical dosage form according to claim 1, which provides modified release of indapamide or the physiologically acceptable salt thereof; preferably prolonged release (retarded release, sustained release), delayed release, or extended release; more preferably prolonged release.

3. The pharmaceutical dosage form according to claim 1 or 2, wherein indapamide is embedded in a modified release matrix comprising a modified release matrix material.

4. The pharmaceutical dosage form according to claim 3, wherein the modified release matrix comprises or essentially consists of at least one polymer selected from the group consisting of
- cellulose ethers; preferably hydroxypropyl cellulose (HPC), hydroxypropyl methyl cellulose (HPMC), hydroxyethyl cellulose (HEC), methyl cellulose (MC), ethyl cellulose (EC), sodium carboxymethyl cellulose and carboxymethyl cellulose;
- cellulose esters; preferably cellulose acetate, cellulose acetate succinate, cellulose acetate phthalate, cellulose acetate propionate, cellulose acetate butyrate;
- acrylic polymers; preferably selected from cross-linked acrylic homopolymers, cross-linked acrylic copolymers, linear acrylic homopolymers, linear acrylic copolymers, cross-linked methacrylic homopolymers, cross-linked methacrylic copolymers, linear methacrylic homopolymers, and linear methacrylic copolymers; preferably selected from poly(acrylic acid), copolymers of acrylic acid, poly(acrylate), acrylate copolymers, poly(methyl acrylate), methyl acrylate copolymers, poly(ethoxyethyl acrylate), ethoxyethyl acrylate copolymers, poly(cyanoethyl acrylate), cyanoethyl acrylate copolymers, poly(aminoalkyl acrylate), aminoalkyl acrylate copolymers, poly(acrylic acid, acrylic acid alkylamide), poly(methacrylic acid, acrylic acid alkylamide), poly(hydroxyethyl acrylate), hydroxyethyl acrylate copolymers, poly(acrylic acid anhydride), acrylic acid anhydride copolymers, poly(acrylamide), acrylamide copolymers, poly(glycidyl acrylate), glycidyl acrylate copolymers, poly(methacrylic acid), methacrylic acid copolymers, poly(methacrylate), methacrylate copolymers, poly(methyl methacrylate), methyl methacrylate copolymers, poly(ethoxyethyl methacrylate), ethoxyethyl methacrylate copolymers, poly(cyanoethyl methacrylate), cyanoethyl methacrylate copolymers, poly(aminoalkyl methacrylate), aminoalkyl methacrylate copolymers, poly(acrylic acid, methacrylic acid alkylamide), poly(methacrylic acid, methacrylic acid alkylamide), poly(hydroxyethyl methacrylate), hydroxyethyl methacrylate copolymers, poly(methacrylic acid anhydride), methacrylic acid anhydride copolymers, poly(methacrylamide), methacrylamide copolymers, poly(glycidyl methacrylate), and glycidyl methacrylate copolymers;
- polysaccharides or gums; preferably xanthan gum, guar gum, karaya gum, locust bean gum, sodium alginate, carob gum, chitosan, polysaccharides of mannose and galactose, pectin, tragacanth, and agar-agar;
- polyalkylene glycols and polyalkylene oxides;
- polyvinylalcohol (PVA), cross-linked polyvinylalcohol (PVA), polyvinylpyrrolidone (PVP), cross-linked polyvinylpyrrolidone (PVP), polyvinylpyrrolidone-vinylacetate copolymers, polyvinylchloride (PVC), polyethylene vinyl acetate (PVA), polydimethylsiloxane (PDS), polyether urethane (PEU), polylactic acid (PLA), polyglycolic acid (PGA), polycaprolactone (PCL), polyanhydrides, polyorthoesters; and
- protein derived materials;
preferably selected from cellulose ethers and acrylic polymers.

5. The pharmaceutical dosage form according to claim 3 or 4, wherein the modified release matrix material comprises or essentially consists of at least one cellulose ether, at least one acrylic polymer, or a mixture of at least one cellulose ether with at least one acrylic polymer; preferably a first cellulose ether, a second cellulose ether, and/or an acrylic polymer; more preferably the first cellulose ether and the acrylic polymer.

6. The pharmaceutical dosage form according to claim 5, wherein the first cellulose ether and the second cellulose ether are independently of one another selected from hydroxypropyl cellulose (HPC), hydroxypropyl methyl cellulose (HPMC) and hydroxyethyl cellulose (HEC); preferably hydroxypropyl methyl cellulose (HPMC).

7. The pharmaceutical dosage form according to claim 5 or 6, wherein the acrylic polymer is a polymer of acrylic acid; preferably a carbomer.

8. The pharmaceutical dosage form according to any of the preceding claims, which comprises at least one disintegrant; preferably selected from the group consisting of crospovidone, starch, maize starch, native starch, pregelatinized starch, sodium starch glycolate, hydroxypropyl starch, microcrystalline cellulose, sodium and/or calcium salts of carboxymethyl cellulose, cross-linked carboxymethylcellulose (e.g. croscarmellose sodium and/or croscarmellose calcium), low substituted hydroxypropyl cellulose, methylcellulose, polacrilin potassium, docusate sodium, alginic acid, sodium and/or calcium alginate, agar, guar gum, chitosan, and combinations thereof; preferably crospovidone.

9. The pharmaceutical dosage form according to any of the preceding claims, which comprises at least one diluent; preferably selected from the group consisting of
- lactose; preferably lactose monohydrate, anhydrous lactose, amorphous lactose, crystalline lactose, and spray dried and/or granulated lactose;
- monosaccharides, disaccharides and oligosaccharides; preferably sucrose, glucose, fructose, dextrates, saccharose, raffinose, trehalose, and dextrin,
- sugar alcohols; preferably xylitol, mannitol, maltitol, isomalt, and sorbitol;
- cellulose or cellulose derivatives; preferably powdered cellulose, microcrystalline cellulose, and silicified microcrystalline cellulose;
- starch or starch derivatives; preferably low moisture starch, corn starch, pregelatinized starch, and low moisture pregelatinized starch; and
- combinations thereof;
preferably lactose, sugar alcohols, cellulose and combinations thereof; more preferably crystalline lactose, mannitol, microcrystalline cellulose and combinations thereof; still more preferably mannitol, microcrystalline cellulose and combinations thereof.

10. The pharmaceutical dosage form according to any of the preceding claims, which comprises at least one glidant; preferably selected from the group consisting of colloidal silica, talc, magnesium trisilicate, and combinations thereof; preferably colloidal silica.

11. The pharmaceutical dosage form according to any of the preceding claims, which comprises at least one lubricant; preferably selected from stearic acid, magnesium stearate, calcium stearate, aluminum stearate, zinc stearate, magnesium palmitate, magnesium oleate, sodium stearyl fumarate, glycerol tribehenate and combinations thereof; preferably magnesium stearate.

12. The pharmaceutical dosage form according to any of the preceding claims, which is selected from the group consisting of tablets, pills, powders, lozenges, sachets, soft gelatin capsules, hard gelatin capsules, and suppositories; preferably tablets; more preferably bilayer tablets.

13. The pharmaceutical dosage form according to any of the preceding claims, wherein the dosage form is a bilayer tablet comprising a first layer which comprises valsartan or the physiologically acceptable salt thereof and a second layer which comprises indapamide or the physiologically acceptable salt thereof.

14. The pharmaceutical dosage form according to any of the preceding claims for use in the treatment or prevention of a condition or disease selected from the group consisting of hypertension, heart failure, such as (acute and chronic) congestive heart failure, left ventricular dysfunction and hypertrophic cardiomyopathy, diabetic cardiac myopathy, supra-ventricular and ventricular arrhythmias, atrial fibrillation, atrial flutter, detrimental vascular re-modeling, myocardial infarction and its sequelae, atherosclerosis, angina (whether unstable or stable), renal insufficiency (diabetic and non-diabetic), angina pectoris, diabetes, secondary aldosteronism, primary and secondary pulmonary hypertension, renal failure conditions, such as diabetic nephropathy, glomerulonephritis, scleroderma, glomerular sclerosis, proteinuria of primary renal disease, and also renal vascular hypertension, diabetic retinopathy, the management of other vascular disorders, such as migraine, peripheral vascular disease, Raynaud's disease, luminal hyperplasia, cognitive dysfunction, such as Alzheimer's, glaucoma and stroke; preferably hypertension and heart failure.

15. A process for the preparation of a pharmaceutical dosage form according to any of the preceding claims, the process comprising the steps of:
(a) granulating valsartan with one or more excipients; preferably at least one diluent, at least one disintegrant, optionally at least one glidant, and optionally at least one lubricant; thereby obtaining a valsartan granulate; optionally sieving the valsartan granulate;
(b) mixing the valsartan granulate obtained in step (a) with one or more extragranular excipients; preferably at least one diluent, and optionally at least one lubricant; thereby obtaining a valsartan compression mixture;
(c) granulating indapamide with a modified release matrix material and one or more excipients; preferably at least one diluent, optionally at least one glidant, and optionally at least one lubricant; thereby obtaining an indapamide granulate; optionally sieving the indapamide granulate;
(d) mixing the indapamide granulate obtained in step (c) with a modified release matrix material and one or more extragranular excipients; preferably at least one diluent, optionally at least one glidant, and optionally at least one lubricant; thereby obtaining an indapamide compression mixture; and
(e) optionally, compressing the valsartan compression mixture obtained in step (b) and the indapamide compression mixture obtained in step (d) by means of a tablet press thereby obtaining a tablet; preferably a bilayer tablet; more preferably a bilayer tablet comprising a first layer containing valsartan or the physiologically acceptable salt thereof and a second layer containing indapamide or the physiologically acceptable salt thereof.
